# EUROPÄISCHE PATENTSCHRIFT

(11) **EP 1 412 346 B1**
(45) Veröffentlichungstag und Bekanntmachung des Hinweises auf die Patenterteilung: **09.03.2005**
(21) Anmeldenummer: 02754847.8
(22) Anmeldetag: 08.07.2002
(51) Int. Cl.: C07D 313/00, C07H 17/08, C12P 19/62

(54) **VERFAHREN ZUM HERSTELLEN VON NEUEN SPINOSYN-DERIVATEN**
METHOD FOR PRODUCING NOVEL SPINOSYN DERIVATIVES
PROCEDE POUR PRODUIRE DE NOUVEAUX DERIVES DE SPINOSYNE

(30) Priorität: 20.07.2001 DE 10135550
(43) Veröffentlichungstag der Anmeldung: 28.04.2004
(73) Patentinhaber: Bayer CropScience AG, 40789 Monheim (DE)
(72) Erfinder: JESCHKE, Peter, 51467 Bergisch Gladbach (DE); EBERZ, Günther, 51519 Odenthal (DE); FRÖDE, Rita, Cary, NC 27511 (US); MÖHRLE, Volker, 50733 Köln (DE); VELTEN, Robert, 51061 Köln (DE)
(86) Internationale Anmeldenummer: PCT/EP2002/007572
(87) Internationale Veröffentlichungsnummer: WO 2003/010155

(56) Entgegenhaltungen:
- WO-A-97/00265

## Beschreibung

Die vorliegende Erfindung betrifft Verfahren zum Herstellen von neuen Spinosyn-Derivaten, die in C-21-Position mit einem 1-Hydroxy-ethyl-Rest substituiert sind, sowie solche neue Spinosyn-Derivate per se und deren Verwendung zum Herstellen neuer Spinosyne.

Bei den Spinosynen handelt es sich um bekannte Verbindungen. Spinosyne sind Fermentationsprodukte, die von Kulturen des Actinomyceten Saccharopolyspora spinosa hergestellt werden. Natürliche Spinosyne bestehen aus einem tetracyclischen Polyketidgrundgerüst (Aglykon) mit einem 12-gliedrigen Makrolidring und einem 5,6,5-cis-anti-trans-Tricyclus, sowie einem D-Forosamin- und einem 2,3,4-Tri-O-methyl-L-rhamnose-Zuckeranteil (Kirst et al., 1991, Tetrahedron Letters, 32:4839). Mehr als 20 verschiedene natürliche Spinosyne, der sogenannte A83543 Komplex, sind bisher beschrieben worden (vgl. WO 97/00265, WO 94/20518 und WO 93/09126). Beispielsweise sind in EP-A 375316 die Spinosyne A, B, C, D, E, F, G, H und J beschrieben. Aus WO 93/09126 sind die Spinosyne L, M, N, Q, R, S und T bekannt. In WO 94/20518 werden die Spinosyne K, O, P, U, V, W und Y sowie deren Derivate erwähnt. Diese Verbindungen variieren in der Substitution von einer oder einigen Methylgruppen am tetracyclischen Grundgerüst, am D-Forosamin- oder am 2,3,4-Tri-O-methyl-L-rhamnose-Zuckeranteil. Ein 17-Pseudoaglykon, dem der D-Forosamin-Zuckeranteil fehlt, ist ebenfalls aus Kulturbrühen von S. spinosa isoliert worden.

Die Hauptkomponenten des von S. spinosa gebildeten A83543 Komplexes stellen die Varianten Spinosyn A und Spinosyn D dar, die die wesentlichen Bestandteile des Produktes Spinosad sind (vgl. Pesticide Manual, British Crop Protection Council, 11^{th} Ed., 1997, Seite 1272 und Dow Elanco Trade Magazine Down to Earth, Vol. 52, NO. 1,1997 und die darin zitierte Literatur).

Wenn der Amino-Zucker in C-17-Position nicht vorhanden ist, werden die Verbindungen als Spinosyn A, D, etc.-17-Pseudoaglykon bezeichnet; wenn der neutrale Zucker in C-9-Position nicht vorhanden ist, werden die Verbindungen als Spinosyn A, D, etc.-9-Pseudoaglykon bezeichnet. Spinosyne ohne die beiden Zucker-Reste in den Positionen C-9 und C-17 werden als Spinosyn-Aglykon bezeichnet.

Spinosyne sind geeignet zur Bekämpfung von Arachniden, Nematoden, Ek-toparasiten (vgl. WO 01/11962, WO 01/11963, WO 01/11964) und Insekten, insbesondere von Lepidopteren und Dipteren-Spezies. Darüber hinaus ist die technische Anwendung der Spinosyne umweltfreundlich und zudem besitzt diese Substanzklasse ein attraktives toxikologisches Profil.

Man kann jedoch erwarten, dass tierische Schädlinge, insbesondere Ektoparasiten oder Pflanzenschädlinge, die zur Zeit mit Spinosynen bekämpft werden, eine Resistenz gegen diese auf dem Markt befindlichen Wirkstoffe entwickeln können. Daher ist es wichtig, neue biologisch aktive Derivate von Spinosynen herzustellen, welche die derzeit zur Bekämpfung von Schädlingen verwendete Spinosyne ersetzen können.

Bestimmte natürliche Aglykon-Derivate von Spinosyn, die eine Hydroxylgruppe in C-8-Position des Makrolidgrundgerüstes besitzen, wurden kürzlich mittels Saccharopolyspora sp. (LW107129) erzeugt und sind als insektizid wirksame Verbindungen bekannt geworden (vgl. WO 01/19840). Während zahlreiche Modifizierungen an Spinosynen durchgeführt wurden (vgl. WO 97/00265) fanden Derivatisierungen an der Methyl- und Ethylgruppe in C-21-Position des Makrolidgrundgerüstes nur wenig Beachtung. Obwohl die Funhrtionalisierung des Alkylrestes in C-21-Position u. a. für Derivatisierungsreaktionen vorteilhaft wäre, sind nur wenige Spinosyn-Derivate, deren Substituenten in C-21 eine Hydroxylgruppe aufweisen, bekannt. Beispielsweise wurden erst kürzlich Spinosyn-Derivate beschrieben, die mit einem 3-Hydroxy-1-butenyl-Rest in C-21 substituiert sind, gegebenenfalls zusätzlich eine Hydroxylgruppe in der oben genannten C-8-Position tragen können, und eine insektizide Wirksamkeit besitzen (vgl. WO 01/19840).

Während chemisch synthetische Verfahren zur Herstellung von Spinosyn-Derivaten beschrieben sind (vgl. Martynow, J. G. und Kirst, H. A., 1994, J. Org. Chem., 59: 1548), ist über die chemische Synthese der oben genannten Spinosyn-Derivate mit einem 1-Hydroxy-ethyl-Rest in C-21-Position bisher nichts bekannt geworden.

Es ist die Aufgabe der vorliegenden Erfindung, geeignete Verfahren bereitzustellen, welche für die selektive und/oder stereospezifische Herstellung von neuen Spinosyn-Derivaten mit einem 1-Hydroxy-ethyl-Rest in C-21-Position verwendbar sind.

Die Aufgabe wurde gelöst durch die Bereitstellung von Verfahren zum Herstellen von Verbindungen der allgemeinen Formel (I), 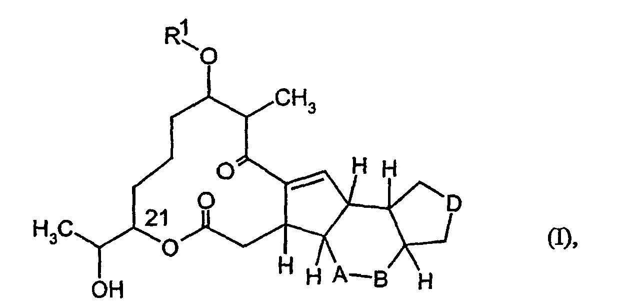 in welcher
- A-B: für eine der folgenden Gruppen steht: -HC=CH-, -HC=C(CH₃)-, -H₂C-CH₂- oder -H₂C-CH(CH₃)-,
- D: für die Gruppe 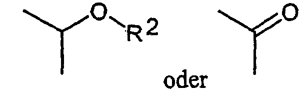 steht,
- R¹: für Wasserstoff oder für einen Amino-Zucker steht, und
- R²: für Wasserstoff oder für einen Zucker steht,
wobei man Verbindungen der allgemeinen Formel (II), 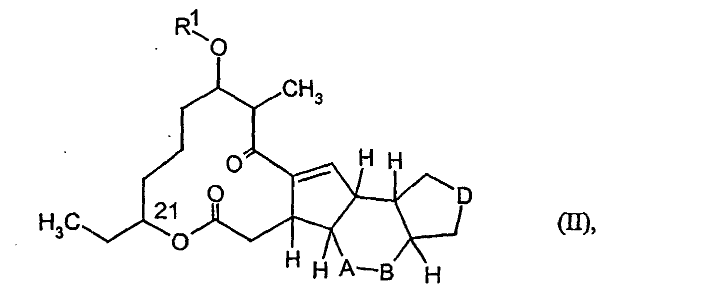 in welcher
A-B, D und R¹ die oben genannten Bedeutungen haben,
mit einem Mikroorganismus in einem wässrigen Nährmedium unter aeroben Bedingungen oder mit einem daraus hergestellten Enzymextrakt oder mit einem oder mehreren daraus isolierten Enzymen in Kontakt bringt.

Die Ausgangsverbindungen werden somit selektiv und/oder stereospezifisch durch Biotransformation unter Verwendung von Mikroorganismen oder deren Enzyme in Spinosyn-Derivate, die in C-21-Position mit einem 1 -Hydroxy-ethyl-Rest substituiert sind, überführt.

Der Ausdruck "Spinosyn-Derivate", wie er hierin verwendet wird, umfasst auch Spinosyn-Aglykon-Verbindungen, d.h. Verbindungen, die das Makrolidgrundgerüst der Spinosyne, jedoch keine Zucker-Reste besitzen.

Bevorzugt werden als Ausgangsverbindungen solche Verbindungen der allgemeinen Formel (II) eingesetzt,
in welcher in dem Fall (1), dass
- A-B: für eine der folgenden Gruppen steht: -HC=CH-, -HC=C(CH₃)-, -H₂C-CH₂- oder -H₂C-CH(CH₃)-, und
- D: für die Gruppe 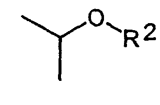 steht,
- R¹: für einen Amino-Zucker der Formel 1a steht, 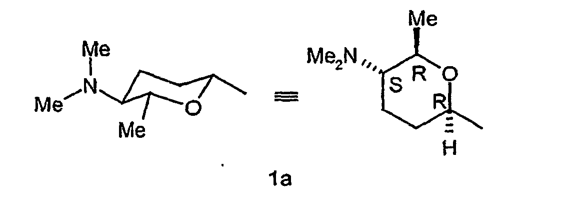 und
- R²: für einen Zucker der Formel 2a steht, 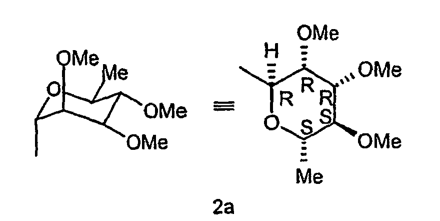
oder in welcher in dem Fall (2), dass
- A-B: für die Gruppe -HC=CH- oder -H₂C-CH₂- steht, und
- D: die oben genannte Bedeutung hat,
- R¹: für einen Aminozucker der oben genannten Formel 1a steht, und
- R²: für Wasserstoff oder für einen Zucker der Formel 2b, 2c, 2d, 2e oder 2f steht, 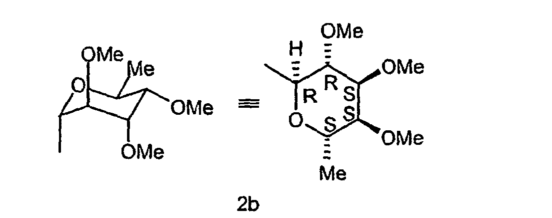 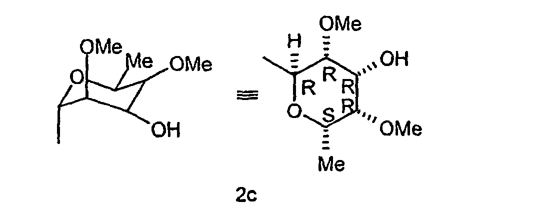 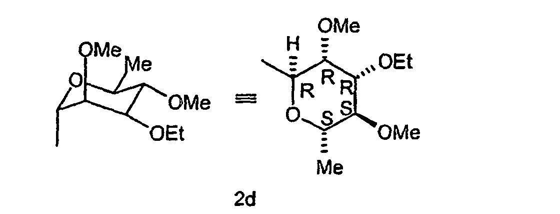 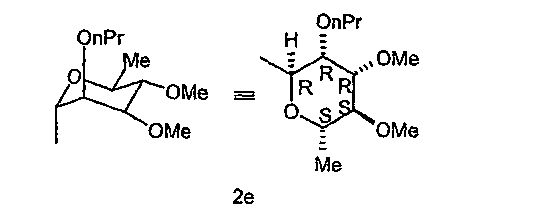 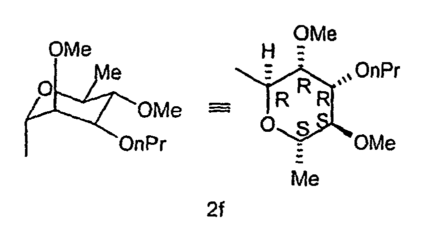
oder in welcher in dem Fall (3), dass
- A-B: für eine der folgenden Gruppen steht: -HC=CH-, -HC=C(CH₃)- oder -H₂C-CH₂-, und
- D: die oben genannte Bedeutung hat,
- R¹: für Wasserstoff oder für einen Amino-Zucker der Formel 1b steht, 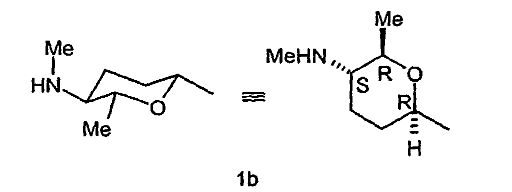 und
- R²: für Wasserstoff oder für einen Zucker der oben genannten Formel 2a steht,
oder in welcher in dem Fall (4), dass
- A-B: für die Gruppe -HC=CH- oder -HC=C(CH₃)- steht, und
- D: die oben genannte Bedeutung hat,
- R¹: für einen Aminozucker der oben genannten Formel 1a steht, und
- R²: für einen Zucker der Formel 2g, 2h, 2i, 2j oder 2k steht, 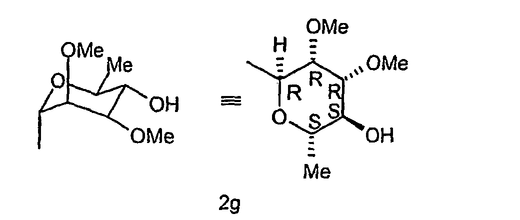 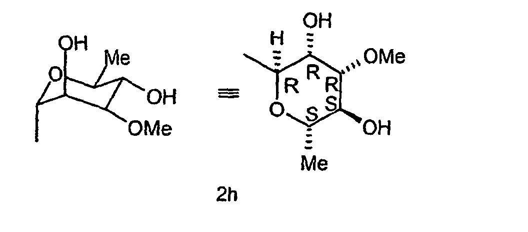 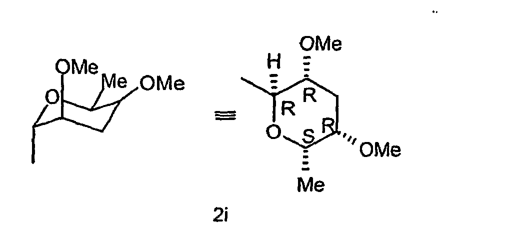 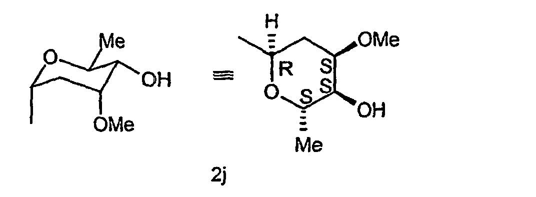 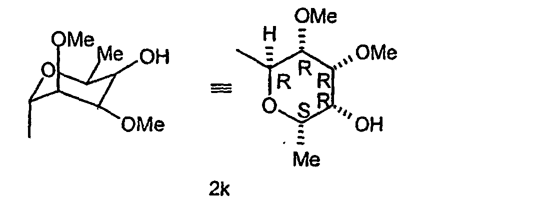
oder in welcher in dem Fall (5), dass
- A-B: für die Gruppe -HC=CH- oder -H₂C-CH₂- steht, und
- D: die oben genannte Bedeutung hat,
- R¹: für einen Aminozucker der oben genannten Formel 1 a steht, und
- R²: für einen Zucker der Formel 21 oder 2m steht, 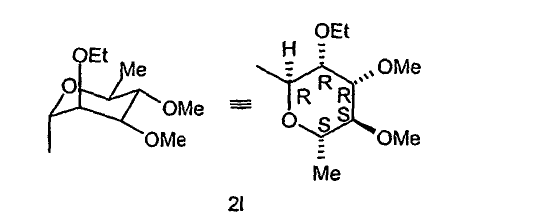 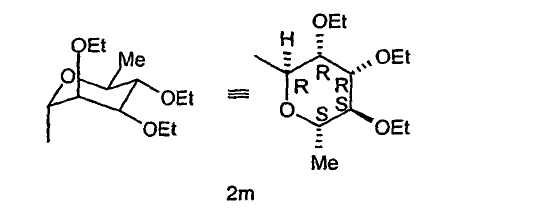
oder in welcher in dem Fall (6), dass
- A-B: für die Gruppe -HC=CH- oder -HC=C(CH₃)- steht, und
- D: die oben genannte Bedeutung hat,
- R¹: für Wasserstoff oder für einen Aminozucker der oben genannten Formel 1b oder für einen Aminozucker der Formel 1c steht, 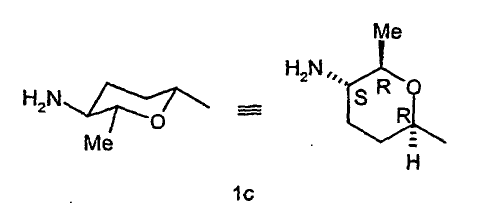 und
- R²: für einen Zucker der oben genannten Formel 2b, 2c, 2g oder 2h, oder für einen Zucker der Formel 2n steht, 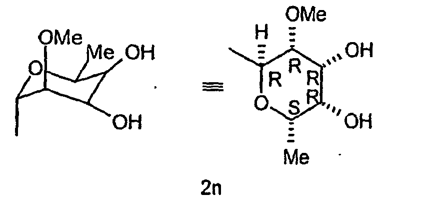
oder in welcher in dem Fall (7), dass
- A-B: für die Gruppe -HC=CH- steht, und
- D: die oben genannte Bedeutung hat,
- R¹: für einen Aminozucker der oben genannten Formel 1a steht, und
- R²: für einen Zucker der Formel 2o steht, 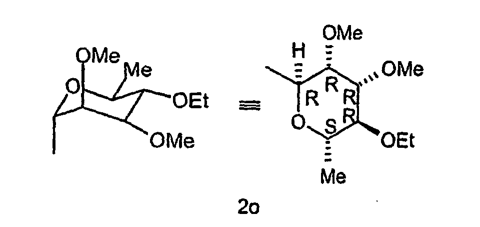
oder in welcher in dem Fall (8), dass
- A-B: für die Gruppe -HC=CH- steht, und
- D: die oben genannte Bedeutung hat,
- R¹: für einen Aminozucker der oben genannten Formel 1b steht, und
- R²: für einen Zucker der oben genannten Formel 2d, 2i oder 2j, oder für einen Zucker der Formel 2p steht, 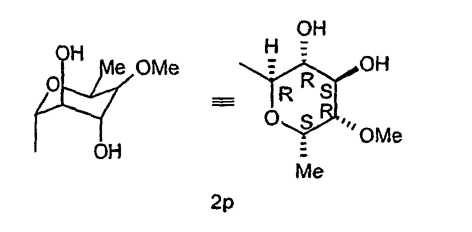
oder in welcher in dem Fall (9), dass
- A-B: für die Gruppe -HC=CH- steht, und
- D: die oben genannte Bedeutung hat,
- R¹: für Wasserstoff oder für einen Aminozucker der oben genannten Formel 1c steht, und
- R²: für einen Zucker der oben genannten Formel 2i oder 2p steht,
oder in welcher in dem Fall (10), dass
- A-B: für die Gruppe -HC=CH- steht, und
- D: die oben genannte Bedeutung hat,
- R¹: für einen Aminozucker der Formel 1d, 1e oder 1f steht, 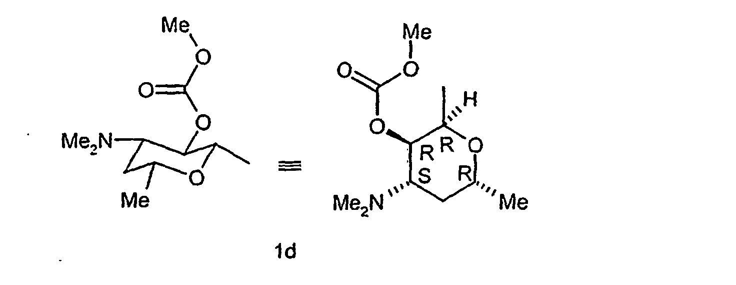 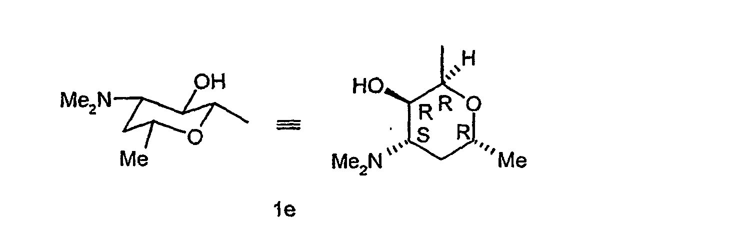 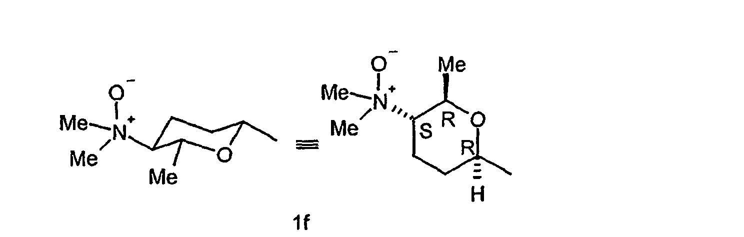 und
- R²: für einen Zucker der oben genannten Formel 2a steht,
oder in welcher in dem Fall (11), dass
- A-B: für die Gruppe -HC=CH- steht, und
- D: für die Gruppe 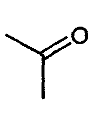 steht,
- R¹: für Wasserstoff oder für einen Aminozucker der oben genannten Formel 1a steht.

Besonders bevorzugt werden als Ausgangsverbindungen solche Verbindungen der allgemeinen Formel (II) eingesetzt,
in welcher in dem Fall (12), dass
- A-B: für die Gruppe -HC=CH- oder -HC=C(CH₃)- steht, und
- D: für die Gruppe 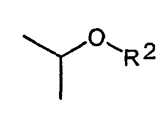 steht,
- R¹: für einen Amino-Zucker der Formel 1a steht, und
- R²: für einen Zucker der Formel 2a, 2g oder 2h steht,
oder in welcher in dem Fall (13), dass
- A-B: für die Gruppe -HC=CH- steht, und
- D: die oben genannte Bedeutung hat,
- R¹: für einen Aminozucker der Formel 1a steht, und
- R²: für Wasserstoff oder für einen Zucker der Formel 2d, 2e, 21, 2m oder 2o steht,
oder in welcher in dem Fall (14), dass
- A-B: für die Gruppe -HC=CH- oder -HC=C(CH₃)- steht, und
- D: die oben genannte Bedeutung hat,
- R¹: für Wasserstoff oder für einen Amino-Zucker der Formel 1b steht, und
- R²: für Wasserstoff oder für einen Zucker der Formel 2a steht,
oder in welcher A-B, D und R¹ wie im Fall (11) definiert sind.

Ganz besonders bevorzugt werden als Ausgangsverbindungen Verbindungen der allgemeinen Formel (II) eingesetzt,
in welcher in dem Fall (15), dass
- A-B: für die Gruppe -HC=CH- oder -HC=C(CH₃)- steht, und
- D: für die Gruppe 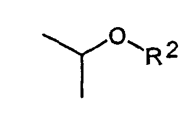 steht,
- R¹: für einen Amino-Zucker der Formel 1a steht, und
- R²: für einen Zucker der Formel 2a steht,
oder in welcher in dem Fall (16), dass
- A-B: für die Gruppe -HC=CH- steht, und
- D: die oben genannte Bedeutung hat,
- R¹: für einen Aminozucker der Formel 1a steht, und
- R²: für Wasserstoff oder für einen Zucker der Formel 2d, 2l oder 2m steht,
oder in welcher A-B, D und R¹ wie im Fall (11) definiert sind.

Am meisten bevorzugt werden als Ausgangsverbindungen Verbindungen der allgemeinen Formel (II) eingesetzt,
in welcher in dem Fall (17), dass
- A-B: für die Gruppe -HC=CH- oder -HC=C(CH₃)- steht, und
- D: für die Gruppe 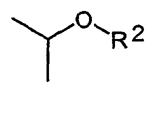 steht,
- R¹: für einen Amino-Zucker der Formel 1a steht, und
- R²: für einen Zucker der Formel 2a steht,
oder in welcher in dem Fall (18), dass
- A-B: für die Gruppe -HC=CH- steht, und
- D: die oben genannte Bedeutung hat,
- R¹: für Wasserstoff steht, und
- R²: für Wasserstoff steht.

Gegenstand der vorliegenden Erfindung sind auch die Verbindungen der allgemeinen Formel (I), worin A-B, D und R¹ die oben genannten Bedeutungen haben.

Die hierin verwendete Abkürzung "Me" steht für Methyl; die Abkürzung "Et" steht für Ethyl.

Durch das erfindungsgemäße Verfahren können sowohl die optisch aktiven, stereoisomeren Formen, aber auch diastereomere Formen der Verbindungen der allgemeinen Formel (I) gebildet werden.

Die erfindungsgemäßen Verbindungen der Formel (I) können in stereoisomeren Formen, die sich entweder wie Bild und Spiegelbild (Enantiomere), oder die sich nicht wie Bild und Spiegelbild (Diastereomere) verhalten, existieren. Gegenstand der vorliegenden Erfindung sind sowohl die Enantiomeren als auch die Diastereomeren und deren jeweilige Mischungen. Die Racemformen lassen sich ebenso wie die Diastereomeren in bekannter Weise in die stereoisomer einheitlichen Bestandteile trennen. Gegebenenfalls lassen sich die Isomere durch an sich bekannte Verfahren ineinander überführen.

Die erfindungsgemäßen Verbindungen, in welcher R¹ für einen Aminozucker der Formeln 1a - 1e steht, können Salze bilden. Salze werden gemäß den Standardverfahren zur Salzherstellung gebildet. Beispielsweise werden die erfindungsgemäßen Verbindungen mit entsprechenden Säuren neutralisiert um Säureadditionssalze auszubilden. Repräsentativ verwendbare Säureadditionssalze sind Salze, die sich beispielsweise durch eine Reaktion mit anderen anorganischen Säuren, wie beispielsweise Schwefelsäure, Salzsäure, Bromwasserstoffsäure, Phosphorsäure oder organischen Carbonsäuren wie Essigsäure, Trifluoressigsäure, Zitronensäure, Bernsteinsäure, Milchsäure, Ameisensäure, Maleinsäure, Camphersäure, Phthalsäure, Glycolsäure, Glutarsäure, Stearinsäure, Salicylsäure, Sorbinsäure, Zimtsäure, Pikrinsäure, Benzoesäure oder organischen Sulfonsäuren wie Methansulfonsäure und para-Toluolsulfonsäure oder mit basischen Aminosäuren wie Asparaginsäure, Glutaminsäure, Arginin oder Ähnliches bilden.

Die als Ausgangsverbindungen für das erfindungsgemäße Verfahren verwendbaren Verbindungen der allgemeinen Formel (II) sind bekannt (vgl. Creemer L. C. et al., 1998, J. Antibiotics 51 (8): 795-800; Sparks T. C. et al., 1998, J. Econ. Entomol. 91 (6): 1277-1283; Sparks T. C. et aL, 2000, Pestic. Biochem. Physiol. 67 (3): 187-197; Paquette L. A. et al., 1998, J. Am. Chem. Soc. 120 (11): 2553-2563; Evans D. A. et al., 1993, J. Am. Chem. Soc. 115 (11): 4497-4513; Crouse G. D. et al., 2001, Pest. Manag. Sci. 57 (2): 177-185; Creemer L. C. et al., 2000, J. Antibiotics 53 (2): 171-178; Sparks T. C. et al., 2000, Proc.-Beltwide Cotton Conf. Vol. 2: 1225-1229) bzw. können nach dem in WO 01/16303 beschriebenen Verfahren hergestellt werden. In analoger Weise können die für das erfindungsgemäße Verfahren verwendbaren Ausgangsverbindungen ausgehend von den entsprechenden natürlichen Spinosynen erhalten werden.

Setzt man beispielsweise das Spinosyn A-Aglykon der Formel (IIa) ein, so läßt sich das erfindungsgemäße Verfahren durch das Reaktionsschema 1 wiedergeben:

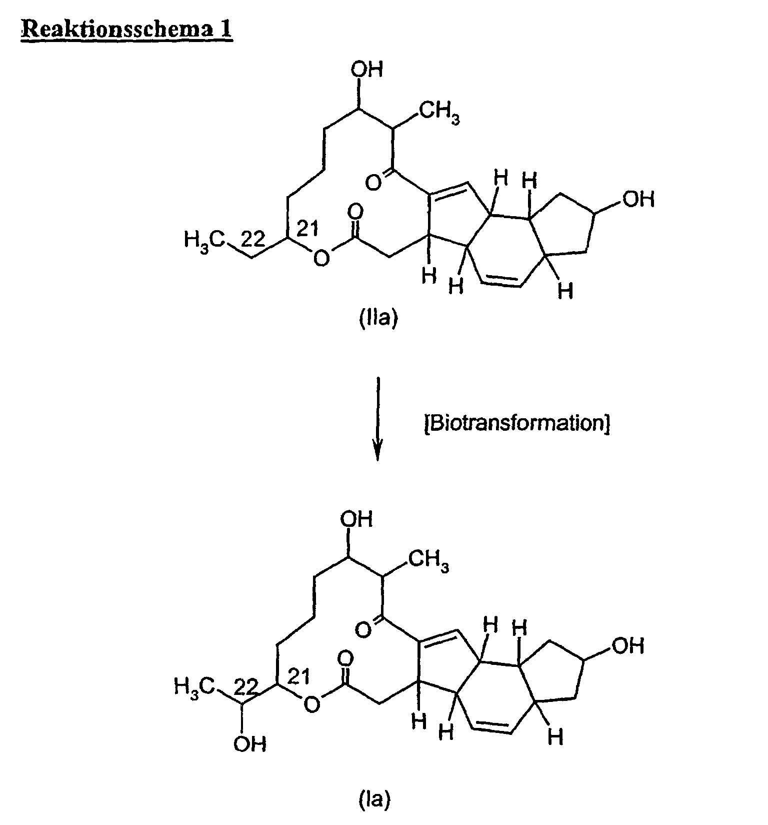

Selektive und/oder stereospezifische Hydroxylierungen natürlicher Produkte und synthetischer Verbindungen durch Biokonversion unter Verwendung von Mikroorganismen oder deren Enzyme sind in der Literatur beschrieben. Insbesondere wurden Zellen und/oder Enzyme von Gram-positiven Bakterien, wie Streptomyceten, oder Gram-negative Bakterien, wie Pseudomonas, oder Pilzen, wie Fusarium, verwendet. Beispiele für Mikroorganismen, die entsprechende Enzym-Klassen, wie beispielsweise P-450-Monooxygenasen enthalten, sind in der folgenden Tabelle aufgelistet:

| **Organismus/Enzym** | **Klasse der chemischen Verbindung** | **Referenz** |
|---|---|---|
| Streptomyces halstedii | Hydroxylierung von Galbonoliden A and B | US 5972994 |
| Streptomyces sp. MA 7065 | Hydroxylierung von Taxol und Cephalomanninen | US 5756536 |
| Streptomyces roseochromogenes (Waksman collection 3689), Streptomyces sp. (ATCC 11009), and Streptomyces roseochromogenes (ATCC 3347) | Hydroxylierung von 11-Oxo- oder 11β-Hydroxy-6α-methylprogesteron zum 16α-Hydroxy-Derivat, welches dann acetyliert wird | US 2864837 |
| Nocardioides luteus | 6-Hydroxy-7-desoxytaxane | US 6162622 |
| Fusarium moniliforme | Herstellung von 7α-Hydroxyl-Derivaten von Dehydroepiandrosteron und Pregnenolon | FR-A 2771105 |
| Beauveria bassiana | 2-Phenoxypropionsäure | WO 95/29249 |
| Cunninghamella blakesleena (ATCC 8688a) | Hydroxylierung von Avermectin | US 4666937 EP-A 194125 |
| Pseudomonas testosteroni ATCC 31492 | m-Hydroxybenzoat wird transformiert zu 2,3-Dihydroxybenzoat | US 4217416 |
| Mortierella maculata | Herstellung von Pravastatin ausgehend von Compactin | WO 00/46175 |
| Pflanzliche NADPH Cytochrom-P450-Reduktase | Hydroxylierung von verschiedenen Substraten | WO 93/21326 |
| Bacterium NRRL-B-18737 | Herstellung von Bisphenolalkyl-alkoholen ausgehend von Bisphenol-alkanen | US 5132228 |
| Pseudomonas mendocina kr-1 Monooxygenase-Gene | Biokonversion einer Phenylverbindung zu einer phenolischen Verbindung | WO 89/09828 |
| Mikroorganismen oder Enzyme | Herstellung optisch aktiver 3-Hydroxy-pyrrolidine | WO 00/29606 |
| Rekombinante Yarrowia lipolytica, die heterologe Cytochrom-P450-Systeme exprimieren | Biokonversion von Progesteron zu 17α-Hydroxyprogesteron | WO 00/03008 |
| Geranylgeraniol-18-hydroxylase, gereinigt aus Croton sublyratus | Biokonversion von Geranylgeraniol zu Plaunotol | US 5879916 |

Erfindungsgemäß und entsprechend dem oben genannten Reaktionsschema 1 kann man Verbindungen der allgemeinen Formel (II), worin A-B, D und R¹ die oben angegebenen Bedeutungen haben, mit einem geeigneten Mikroorganismus in einem wässrigen Nährmedium unter aeroben Bedingungen in Kontakt bringen und danach die gewünschten Verbindungen der allgemeinen Formel (I) isolieren.

Anstelle der Mikroorganismen können auch Enzymextrakte und aufgereinigte Enzyme, gegebenenfalls nach Zugabe oder unter Regenerierung der benötigten Cofaktoren, verwendet werden, die nach üblichen Verfahren ausgehend von diesen Mikroorganismen erhältlich sind.

In besonderer Weise können Gene, die die Biosynthese solcher Enzyme determinieren auch kloniert und in fremden Wirten, wie z.B. Escherichia coli exprimiert werden. Solche rekombinanten Bakterien können unmittelbar zur Biotransformation eingesetzt werden. Darüber hinaus kann auch ein Enzymextrakt einer solchen rekombinanten Zelle oder ein auf gereinigtes Protein, gegebenenfalls nach Zugabe oder unter Regenerierung der benötigten Cofaktoren, zur Biotransformation verwendet werden.

Vorzugsweise wird für das erfindungsgemäße Verfahren ein Mikroorganismus aus der Gruppe der Actinomyceten, insbesondere aus der Gattung Streptomyces verwendet.

Besonders bevorzugt wird für das erfindungsgemäße Verfahren ein Stamm aus der Art Streptomyces djakartensis, Streptomyces griseofuscus, Streptomyces caelestis, Streptomyces antibioticus, Streptomyces griseus oder Streptomyces aureofaciens eingesetzt.

Ganz besonders bevorzugt wird für das erfindungsgemäße Verfahren ein Stamm mit den kennzeichnenden Merkmalen der folgenden Stämme verwendet:

| **Name** | **Hinterlegungs-Nr.** |
|---|---|
| Streptomyces djakartensis | NRRL B-12103 |
| Streptomyces griseofuscus | DSM 40191 |
| Streptomyces caelestis | DSM 40084 |
| Streptomyces antibioticus | ATCC 11891 |
| Streptomyces griseus | DSM 40937 |
| Streptomyces aureofaciens | DSM 46447 |

Die in der Tabelle genannten Stämme sind nochmals bei der Deutschen Sammlung von Mikroorganismen und Zellkulturen GmbH (DSMZ), Mascheroder Weg 1b, D-38124 Braunschweig, Deutschland, in Übereinstimmung mit den Bestimmungen des Budapester Vertrages hinterlegt worden:

| **Name** | **Hinterlegungs-Nr.** |
|---|---|
| Streptomyces djakartensis | DSM 14327 |
| Streptomyces griseofuscus | DSM 14330 |
| Streptomyces caelestis | DSM 14328 |
| Streptomyces antibioticus | DSM 14329 |
| Streptomyces griseus | DSM 14331 |
| Streptomyces aureofaciens | DSM 14332 |

Die Stämme sind in Beispiel 16 näher beschrieben. Es können nicht nur die hinterlegten Stämme als solche, sondern auch Mutanten davon verwendet werden, solange diese Mutanten die kennzeichnenden Merkmalen der hinterlegten Stämme besitzen. Dies bedeutet, dass die Mutanten noch die Fähigkeit besitzen müssen, die erfindungsgemäße Biokonversion durchführen zu können.

Vorzugsweise enthält das wässrige Nährmedium eine assimilierbare Kohlenstoffquelle und eine assimilierbare Stickstoffquelle.

Die Verbindungen der Formel (I) werden beispielsweise produziert, wenn ein Stamm aus den Arten Streptomyces djakartensis, S. griseofuscus, S. caelestis, S. antibioticus, S. griseus oder S. aureofaciens in einem wässrigen Nährmedium unter aeroben Bedingungen in der Gegenwart von Verbindungen der Formel (II) fermentiert werden. Typischerweise werden die Mikroorganismen in einem Nährmedium, welches eine Kohlenstoffquelle und gegebenenfalls ein proteinartiges Material enthält, fermentiert. Bevorzugte Kohlenstoffquellen umfassen Glucose, braunen Zucker, Saccharose, Glycerin, Stärke, Maisstärke, Lactose, Dextrin, Melasse etc. Bevorzugte Stickstoffquellen umfassen Baumwollsaatmehl, Hefe, autolysierte Bäckerhefe, feste Milchbestandteile, Sojabohnenmehl, Maismehl, pankreatische oder papainisch verdaute Spaltprodukte von Kasein, feste Destillationsbestandteile, Brühen aus tierischem Pepton, Fleisch- und Knochenstücke etc. Vorzugsweise werden Kombinationen dieser Kohlenstoff- und Stickstoffquellen verwendet, Spurenelemente, wie beispielsweise Zink, Magnesium, Mangan, Kobalt, Eisen etc. müssen dem Kultivierungsmedium nicht zugefügt werden, solange Leitungswasser und nicht-gereinigte Bestandteile als Mediumkomponenten verwendet werden.

Die Herstellung der Verbindungen der allgemeinen Formel (I) kann bei jeder Temperatur induziert werden, die ein ausreichendes Wachstum der Mikroorganismen gewährleistet. Vorzugsweise beträgt die Temperatur zwischen 21°C und 32°C, besonders bevorzugt ungefähr 28°C. Im allgemeinen wird eine optimale Produktion der Verbindungen der Formel (I) innerhalb von 2 bis 4 Tagen nach Zugabe der Verbindungen der Formel (II) zu der Kultur erhalten. Die Produktion der erfindungsgemäßen Verbindungen kann sowohl in Schüttelflaschen als auch in Rühr-Fermentern stattfinden.

Bevorzugte Anzuchtbedingungen und Medien für Anzuchten im Schüttelkolben sind in den Beispielen 2 bis 9 beschrieben.

Die erfindungsgemäßen Verbindungen als Biotransformationsprodukt können aus dem Kultivierungsmedium durch übliche Verfahren isoliert werden.

Verschiedene Verfahren können angewendet werden, um die erfindungsgemäßen Verbindungen aus der Fermentationsbrühe zu isolieren und zu reinigen, wie z.B. präparative Gelchromatographie, präparative Chromatographie an Reversed Phase oder präparative Absorptionschromatographie. Die Detektion kann dabei z.B. durch UV-Absorption oder Massenspektrometrie erfolgen.

Die erfindungsgemäßen Verbindungen können zur Herstellung von biologisch aktiven, insbesondere insektiziden und akariziden, Spinosynen verwendet werden. Die biologische Wirksamkeit bestimmter natürlicher Aglykon-Derivate von Spinosyn, die eine Hydroxylgruppe in C-8-Position des Makrolidgerüstes besitzen, ist kürzlich bekannt geworden (vgl. WO 01119840). Als weitere Beispiele sind die ebenso kürzlich vorbeschriebenen Spinosyn-Derivate mit einem 3-Hydroxy-1-butenyl-Rest in C-21-Position zu nennen. Gegebenenfalls können diese Spinosyn-Derivate auch zusätzlich eine Hydroxylgruppe in einer der oben genannten C-8-Position tragen und ebenfalls eine Insektizide Wirksamkeit besitzen (vgl. WO 01/19840).

Bei der Herstellung weiterer Spinosyn-Derivate aus den erfindungsgemäßen Verbindungen der allgemeinen Formel (I), in welcher R¹ für Wasserstoff und D für die Gruppe C=O oder C-O-R² steht, worin R² Wasserstoff bedeutet, ist von Vorteil, geeignete Schutzgruppen (SG) zu verwenden. Beispielsweise sind als Schutzgruppen (SG) für Hydroxylgruppen, substituierte Methylether und Ether, substituierte Ethylether, substituierte Benzylether, Silylether, Ester, Carbonate oder Sulfonate bekannt (vgl. Greene T. W., Wuts P. G. W. in Protective Groups in Organic Synthesis; John Wiley & Sohns, Inc. 1999, Protection for the hydroxyl group, including 1,2- and 1,3-diols).

Als Schutzgruppen (SG) vom substituierten Methyleiher-Typ sind beispielsweise zu nennen: Methoxymethylether (MOM), Methylthiomethylether (MTM), (Phenyldimethylsilyl)methoxymethylether (SMOM-OR), Benzyloxymethylether (BOM-OR), para-Methoxybenzyloxymethylether (PMBM-OR), para-Nitrobenzyloxymethylether, ortho-Nitrobenzyloxymethylether (NBOM-OR), (4-Methoxyphenoxy)-methylether (p-AOM-OR), Guaiacolmethylether (GUM-OR), tert.-Butoxymethylether, 4-Pentenyl-oxymethylether (POM-OR), Silyloxymethylether, 2-Methoxyethoxy-methylether (MEM-OR), 2,2,2-Trichlorethoxymethylether, Bis(2-chlorethoxy)-methylether, 2-(Trimethylsilyl)ethoxymethylether (SEM-OR), Methoxymethylether (MM-OR).

Als Schutzgruppen (SG) vom substituierten Ethylether-Typ sind beispielsweise zu nennen: 1-Bthoxyethylether (EE-OR), 1-(2-Chlorethoxy)ethylether (Cee-OR), 1-[2-(Trimethylsilyl)ethoxy]ethylether (SEE-OR), 1-Methyl-1-methoxyethylether (MIP-OR), 1-Methyl-1-benzyloxyethylether (MBE-OR), 1-Methyl-1-benzyloxy-2-fluorethylether, 1-Methyl-1-phenoxy-ethylether, 2,2,2-Trichlorethylether, 1,1-Dianisyl-2,2,2-trichlorethylether (DATE-OR), 1,1, 1, 3,3,3-Hexafluor-2-phenyliso-propyl-ether (HIP-OR), 2-Trimethylsilylethylether, 2-(Benzylthio)ethylether, 2-(Phenyl-selenyl)-ethylether. Als weitere Schutzgruppen (SG) vom Ether-Typ sind beispielsweise zu nennen: Tetrahydropyranylether (THP-OR), 3-Brom-tetrahydropyranylether (3-BrTHP-OR), Tetrahydrothiopyranylether, 1-Methoxy-cyclohexylether, 2- und 4-Picolylether, 3-Methyl-2-picolyl-N-oxido-ether, 2-Quinolinylmethylether (Qm-OR), 1-Pyrenylmethylether, Dipenylmethylether (DPM-OR), para,para'-Dinitrobenzhydrylether (RO-DNB), 5-Dibenzosuberylether, Triphenylmethylether (Tr-OR), α-Naphthyldiphenylmethylether, para-Methoxy-phenyldiphenylmethylether (MMTr-OR), Di(para-methoxy-phenyl}phenylmethylether (DMTr-OR), Tri(para-methoxyphenyl)methylether (TMTr-OR), 4-(4'-Brom-phenacyloxy) phenyldiphenylmethylether, 4,4',4"-Tris(4,5-dichlorphihalimido-phenyl)methylether (CPTr-OR), 4,4',4"-Tris(levulinoyloxy-plnenyl)methylether (TLTr-OR), 4,4',4"-Tris(benzoyloxyphenyl)-methylether (TBTr-OR), 4,4'-Dimelhoxy-3"-[N-(imidazolylmethyl)]-tritylether (IDTr-OR), 4,4'-dimethoxy-3"-[N-(imidazolyl-ethyl)carbamoyl]tritylether (IETr-OR), 1,1-Bis(4-methoxy-phenyl)-1'-pyrenylmethylether (Bmpm-OR), 9-Anthrylether, 9-(9-Phenyl)xanthenylether (pixyl-OR), 9-(9-Phenyl-10-oxo)anthryl (Tritylon-Ether). 4-Methoxy-tetrahydropyranylether (MTHP-OR), 4-Methoxy-tetrahydrothiopyranylether, 4-Methoxy-tetrahydrothio-pyranylether-S,S-dioxid, 1-[(2-Chlor-4-methyl)phenyl]-4-methoxypiperidin-4-yl-ether (CTMP-OR), 1-(2-Fluorphenyl)-4-methoxy-piperidin-4-yl-ether (Fpmp-OR), 1,4-Dioxan-2-yl-ether, Tetrahydrofuranyl-ether, Tetrahydrothiofuranylether, 2,3,3a,4,5,6, 7,7a-Octahydro-7,8,8-trimeihyl-4,7-methanbenzofuran-2-yl-ether (RO-MBF), tert.-Butylether, Allylether, Propargylether, para-Chlophenylether, para-Methoxyphenylether, para-Nitrophenylether, 2,4-Dinitro-phenylether (RO-DNP), 2,3,5,6-Tetrafluor-4-(trifluormethyl)-phenylether, Benzyl-ether (Bn-OR). Als Schutzgruppen vom substituierten Benzylether-Typ sind beispielsweise zu nennen: para-Methoxybenzylether (MPM-OR), 3,4-Dimethoxy-benzylether (DMPM-OR), ortho-Nitrobenzylether, para-Nitrobenzylether, para-Halobenzylether, 2,6-Dichlor-benzylether, para-Cyanobenzylether, para-Phenyl-benzylether, 2,6-Difluorbenzylether, para-Aminoacylbenzylether (PAB-OR), para-Azidobenzylether (Azb-OR), 4-Azido-3-chlorbenzylether, 2-Trifluormethylbenzyl-ether, para-(Methylsulfinyl)benzylether (Msib-OR). Als Schutzgruppen (SG) vom Silyl-Ether-Typ sind beispielsweise zu nennen: Trimethylsilylether (TMS-OR), Triethylsilylether (TES-OR), Triiso-propylsilylether (TIPS-OR), Dimethylisopropylsilylether (IPDMS-OR), Diethylisopropylsilylether (DEIPS-OR), Dimethylhexylsilyl-ether (TDS-OR), tert.-Butyldimethylsilylether (TBDMS-OR), tert.-Butyldiphenylsilylether (TBDPS-OR), Tribenzylsilylether, Tri-para-xylylsilylether, Triphenylsilylether (TPS-OR), Diphenylmethylsilylether (DPMS-OR), Di-tert.butylmethylsilylether (DTBMS-OR), Tris(trimethylsilyl)silylether (Sisylether), (2-Hydroxystyryl)-dime-thylsilylether (HSDMS-OR), (2-Hytiroa-ystyryl)düsopropylsilylether (HSDIS-OR), tert-Butylmethoxyphenylsilylether (TBMPS-OR), tert.-Butoxydiphenylsilylether (DPTBOS-OR). Als Schutzgruppen (SG) vom Ester-Typ sind beispielsweise zu nennen: Formiatester, Benzoylfonmiatester, Acetatester (RO-Ac), Chloracetatester, Dichloracetatester, Trichloracetatester, Trifluoracetatester (RO-TFA), Methoxy-acetatester, Triphenylmethoxyacetatester, Phenoxyacetatester, para-Chlorphenoxy-acetatester, Phenylacetatester, Diphenylacetatester (DPA-OR), Nicotinatester, 3-Phenylpropionatester, 4-Pentenoatester, 4-Oxopentanoatester (Levulinate) (Lev-OR), 4,4-(Ethylendithio)-pentanoatester (RO-LevS), 5-[3-Bis(4-methoxyphenyl)hydroxy-methylphenoxy]-levulinatester, Pivaloatester (Pv-OR), 1-Adamantanoatester, Crotonatester, 4-Methoxy-crotonatester, Benzoatester (Bz-OR), para-Phenyl-benzoatester, 2,4,6-Trimethylbenzoatester (Mesitoate), 4-(Methylthiomethoxy)-butyratester (MTMB-OR), 2-(Methylthiomethoaymethyl)benzoatester (MTMT-OR). Als Schutzgruppen (SG) vom Ester-Typ sind beispielsweise zu nennen: Methylcarbonat, Methoxymethylcarbonat, 9-Fluorenylmethylcarbonat (Fmoc-OR), Ethylcarbonat, 2,2,2-Trichlorethylcarbonat (Troc-OR), 1,1-Dimethyl-2,2,2-trichlor-ethylcarbonat (TCBOC-OR), 2-(Trimethyisilyl)ethylcarbonate (TMSEC-OR), 2-(Phenylsulfonyl)-ethylcarbonate (Psec-OR), 2-(Triphenylphosphonio)-ethylcarbonate (Peoc-OR), tert.-Butylcarbonat (Boc-OR), Isobutylcarbonat, Vinylcarbonat, Allylcarbonat (Alloc-OR), p-Nitrophenylcarbonat, Benzylcarbonat (Z-OR), para-Methoxybenzylcarbonat, 3,4-Dimethoxybenzylcarbonat, ortho-Nitrobenzylcarbonat, para-Nitrobenzylcarbonat, 2-Dansylethylcarbonat (Dnseoc-OR), 2-(4-Nitrophenyl)-cinylcarbonat (Npeoc-OR), 2-(2,4-Dinitrophenyl)ethylcarbonat (Dnpeoc-OR). Als Schutzgruppen (SG) vom Sulfonat-Typ sind beispielsweise zu nennen: Allylsulfonat (Als-OR), Methansulfonate (Ms-OR), Benzylsulfonate, Tosylate (Ts-OR), 2-[(4-Nitrophenyl)ethyl]sulfonate (Npes-OR).

Bei der Herstellung weiterer Spinosyn-Derivate aus den oben genannten Verbindungen der allgemeinen Formel (I) kann es durchaus vorteilhaft sein, dass zunächst der 1-Hydroxy-ethyl-Rest in C-21-Position und gegebenenfalls die Hydroxylgruppe in C-9-Position (im Falle R² = H) mit geeigneten, beispielsweise mit einer der oben genannten Schutzgruppen (SG), blockiert wird. Dabei ist die Verwendung von zwei unterschiedlichen Schutzgruppen (SG¹ bzw. SG²) empfehlenswert, wobei diese entsprechend kompatibel sein sollten, d. h. sich selektiv und unabhängig von einander abspalten lassen. Anschließend kann eine Derivatisierung, beispielsweise eine Glycosidierung mittels chemischer Synthese oder durch mikrobielle Biokonversion (vgl. auch US 5539089; Einführung der Reste R oder R') zu den Spinosyn-Derivaten (Ia-2 oder Ib-2), an der Hydroxylgruppe in der C-17-Position erfolgen (vgl. Schema 2 und 3). 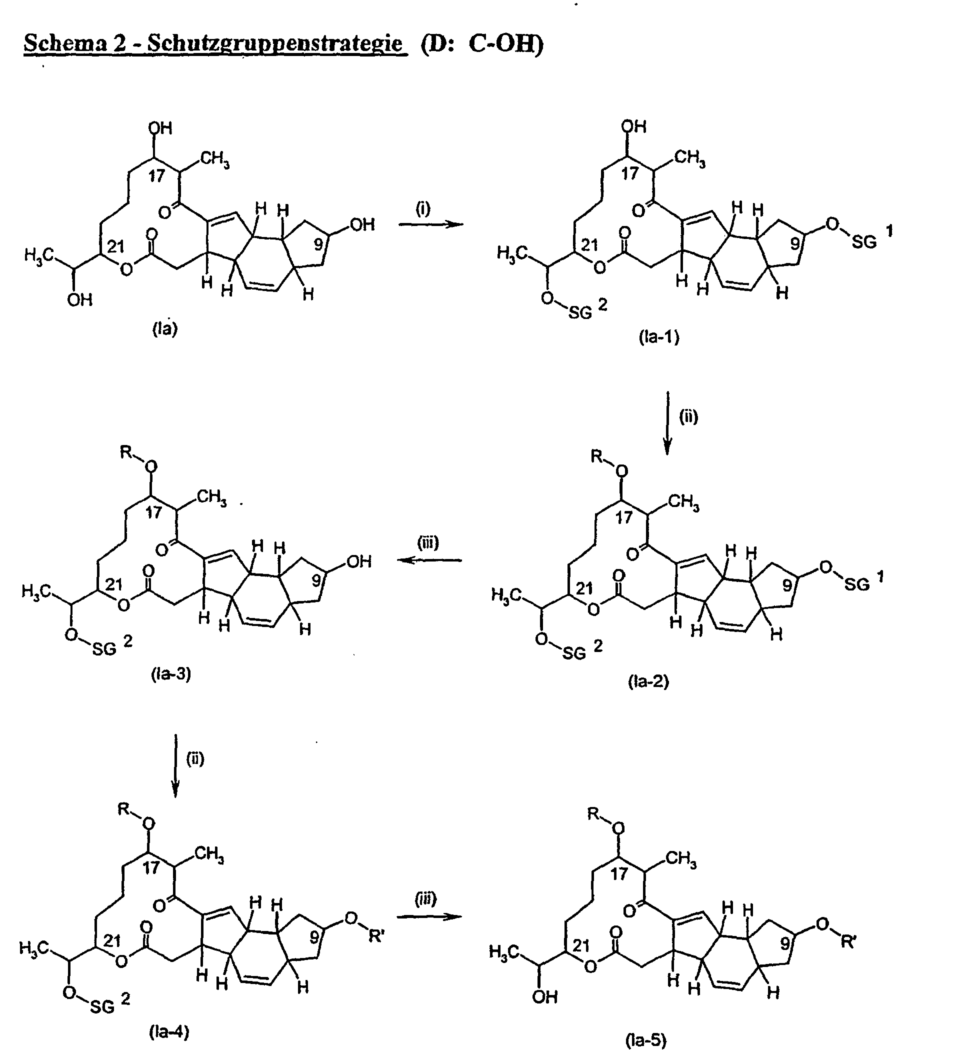
(i) Einführung - Schutzgruppe (SG¹, SG²)
(ii) Derivatisierung- z. B. Glycosidierung (R bzw. R')
(iii) Deblockierung- Schutzgruppe (SG) / oder selektive Deblockierung - Schutzgruppe (z. B. SG¹)

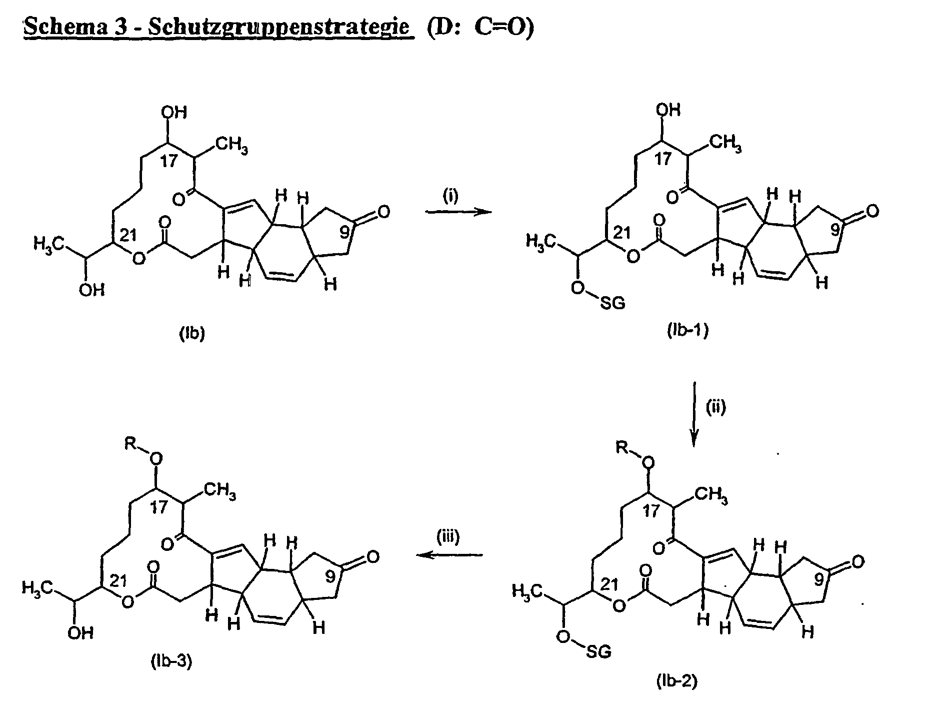

(i) Einführung - Schutzgruppe (SG)
(ii) Derivatisierung - z. B. Glycosidierung (R)
(iii) Deblockierung - Schutzgruppe (SG)

Nach erfolgreicher Derivatisierung in den Positionen C-9 bzw. C-17 (vgl. Ia-4) oder C-17 (vgl. Ib-2) kann die noch vorhandene Schutzgruppe (SG) abgespalten und das Spinosyn-Derivat der allgemeinen Formel (I) erhalten werden.

### Beispiele

### Beispiel 1

### Verwendete Stämme

**Tabelle:**

| | | |
|---|---|---|
| Stämme, die zur Biotransformation der Verbindung (IIa) zu dem entsprechenden Derivaten mit einem 1-Hydroxy-ethyl-Rest in C-21 Position (Verbindung (Ia)) befähigt sind: | | |

| **Name** | **Interne Bezeichnung** | **Hinterlegungs- Nr.** |
|---|---|---|
| Streptomyces djakartensis | NRRL B-12103 | DSM 14327 |
| Streptomyces griseofuscus | DSM 40191 | DSM 14330 |
| Streptomyces caelestis | DSM40084 | DSM 14328 |
| Streptomyces antibioticus | ATCC 11891 | DSM 14329 |
| Streptomyces griseus Streptomyces aureofaciens | DSM 40937 | DSM 14331 |
| | DSM 46447 | DSM 14332 |

ATCC= American Type Culture Collection, Manassas, VA, U.S.A.
DSM= Deutsche Sammlung für Mikroorganismen und Zellkulturen, Braunschweig, Deutschland.
NRRL= Agricultural Research Service Culture Collection, Peoria, IL, U.S.A.

### Beispiel 2

### Biotransformation mit S. djakartensis NRRL B-12103

Beispielhaftes Protokoll der Biotransformation für die Herstellung der Verbindung (Ia) aus der Verbindung (IIa).

Zur Herstellung der Produktionskulturen wurden 20 ml RSA-Medium (RSA-Medium pro Liter: 103 g Sucrose; 0,25 g K₂SO₄ 10,12 g MgCl₂; 10 g Glucose; 5 g Hefeextrakt; 0,1 g Casaminosäuren, 21 g MOPS-Puffer pH 6,8 (KOH), 2 ml Spurenelementlösung; Spurenelementlösung: (pro Liter) 40 mg ZnCl₂, 200 mg FeCl₃ x 6 H₂O, 10 mg CuCl₂ x 2H₂O, 10 mg MnCl₂ x 4 H₂O, 10 mg Na₂B₄O₇ x 10 H₂O, 10mg (NH₄)₆Mo₇O₂₄ x 4 H₂O; (Fernandez et al., 1998, J. Bacteriol. 180: 4929; Hopwood et al., 1985, Genetic manipulation of Streptomyces. A laboratory manual. The John Innes Foundation, Norwich, England) in 100ml-Erlenmeyerkolben mit je 50 µl Sporensuspension von S. djakartensis NRRL B-12103 inokuliert. Vor dem Beimpfen wurde das Medium bei 121°C und 1,1 bar Überdruck für 20 Minuten sterilisiert. Die Kulturen wurden bei 28°C und 200 Upm inkubiert. Nach 24 Stunden sowie nach 72 Stunden Inkubation wurden je 1mg der Verbindung (IIa) (100 µl einer Stammlösung von 10 mg/ml in Methanol) zugegeben. Die Biotransformation wurde nach 120 Stunden abgebrochen. Die Kulturen wurden abzentrifugiert (10 Minuten, 4000 Upm) und der Überstand mit dem gleichen Volumen Methanol versetzt.

### Beispiel 3

### Biotransformationen mit Streptomyces griseofuscus

Beispielhaftes Protokoll einer Biotransformation für die Herstellung der Verbindung (Ia) aus der Verbindung (IIa).

Zur Herstellung der Produktionskulturen wurde das Verfahren von Beispiel 2 unter Verwendung von 50 µl Sporensuspension des Stammes Streptomyces griseofuscus anstelle des Stammes S. djakartensis NRRL B-12103 angewendet.

### Beispiel 4

### Biotransformationen mit Streptomyces caelestis

Beispielhaftes Protokoll einer Biotransformation für die Herstellung der Verbindung (Ia) aus der Verbindung (IIa).

Zur Herstellung der Produktionskulturen wurde das Verfahren von Beispiel 2 unter Verwendung von 50 µl Sporensuspension des Stammes Streptomyces caelestis anstelle des Stammes S. djakartensis NRRL B-12103 angewendet.

### Beispiel 5

### Biotransformationen mit Streptomyces antibioticus

Beispielhaftes Protokoll einer Biotransformation für die Herstellung der Verbindung (Ia) aus der Verbindung (IIa).

Zur Herstellung der Produktionskulturen wurde das Verfahren von Beispiel 2 unter Verwendung von 50 µl Sporensuspension des Stammes Streptomyces antibioticus anstelle des Stammes S. djakartensis NRRL B-12103 angewendet.

### Beispiel 6

### Biotransformationen mit Streptomyces griseus

Beispielhaftes Protokoll einer Biotransformation für die Herstellung der Verbindung (Ia) aus der Verbindung (IIa).

Zur Herstellung der Produktionskulturen wurde das Verfahren von Beispiel 2 unter Verwendung von 50 µl Sporensuspension des Stammes Streptomyces griseus anstelle des Stammes S. djakartensis NRRL B-12103 angewendet.

### Beispiel 7

### Biotransformationen mit Streptomyces aureofaciens

Beispielhaftes Protokoll einer Biotransformation für die Herstellung der Verbindung (Ia) aus der Verbindung (IIa).

Zur Herstellung der Produktionskulturen wurde das Verfahren von Beispiel 2 unter Verwendung von 50 µl Sporensuspension des Stammes Streptomyces aureofaciens anstelle des Stammes S. djakartensis NRRL B-12103 angewendet.

### Beispiel 8

### Biotransformationen mit Streptomyces djakartensis

Beispielhaftes Protokoll einer Biotransformation für die Herstellung von Spinosyn A mit einem 1-Hydroxy-ethyl-Rest in C-21-Position [Verbindung der allgemeinen Formel (I), worin R¹ für einen Amino-Zucker der Formel 1a steht, A-B für die Gruppe -HC=CH- und D für die Gruppe -CO-R² steht, worin R² für einen Zucker der Formel 2a steht].

Zur Herstellung der Produktionskulturen wurden 20 ml RSA-Medium (R5A-Medium: siehe Beispiel 2) in 100 ml-Erlenmeyerkolben mit je 50 µl Sporensuspension von S. djakartensis NRRL B-12103 inokuliert. Vor dem Beimpfen wurde das Medium bei 121°C und 1,1 bar Überdruck für 20 Minuten sterilisiert. Die Kulturen wurden bei 28°C und 200 Upm inkubiert. Nach 48 Stunden Inkubation wurden 2mg Spinosyn A (100 µl einer Stammlösung von 10 mg/ml in Methanol) zugegeben. Die Biotransformation wurde nach 96 Stunden abgebrochen. Die Kulturen wurden abzentrifugiert (10 Minuten, 4000 Upm) und der Überstand mit dem gleichen Volumen Methanol versetzt.

### Beispiel 9

### Biotransformationen mit Streptomyces djakartensis

Beispielhaftes Protokoll einer Biotransformation für die Herstellung von 17-Pseudospinosyn-Aglykon A mit einem 1-Hydroxy-ethyl-Rest in C-21-Position [Verbindung der allgemeinen Formel (I), worin R¹ für Wasserstoff steht, A-B für die Gruppe -HC=CH- oder -HC=C(CH₃)- und D für die Gruppe -CO-R² steht, worin R² für einen Zucker der Formel 2a steht].

Zur Herstellung der Produktionskulturen wurden 20ml R5A-Medium (R5A-Medium: siehe Beispiel 2) in 100 ml-Erlenmeyerkolben mit je 50 µl Sporensuspension von S. djakartensis NRRL B-12103 inokuliert. Vor dem Beimpfen wurde das Medium bei 121°C und 1,1 bar Überdruck für 20 Minuten sterilisiert. Die Kulturen wurden bei 28°C und 200 Upm inkubiert. Nach 48 Stunden Inkubation wurden 2mg 17-Pseudo-spinosyn Aglykon A oder D (100 µl einer Stammlösung von 10mg/ml in Methanol) zugegeben. Die Biotransformation wurde nach 96 Stunden abgebrochen. Die Kulturen wurden abzentrifugiert (10 Minuten, 4000 Upm) und der Überstand mit dem gleichen Volumen Methanol versetzt.

### Beispiel 10

### Isolierung der Verbindung (Ia) aus der Biotransformation mit S. djakartensis NRRL B-12103

Beispielhaftes Protokoll für die Aufarbeitung des Kulturüberstände und Anreicherung der Verbindung (Ia).

35 ml des mit Methanol versetzten Kulturüberstandes aus Beispiel 2 wurden auf etwa 20ml eingeengt und mit 10 ml Wasser versetzt. Es wurde zweimal mit je 10 ml Essigsäureethylester extrahiert, die vereinigten organischen Phasen zur Trockenen eingeengt und der Rückstand in 400 µl Methanol aufgenommen. Ein Aliquot dieser Lösung wurde per HPLC/MS (Beispiel 11) analysiert.

### Beispiel 11

### Analytische HPLC/UV/MS

Beispielhaftes Protokoll für die Analyse der aufgearbeiteten Kulturüberstände mittels HPLC/UV/MS

Ein Aliquot des aufgearbeiteten Kulturüberstands der Biotransformation mit S. djakartensis (Beispiel 2) wurden an einer Reversed-Phase-HPLC-Säule (2,1 x 250 mm) mit einem Gradienten aus Wasser, versetzt mit Ammoniumacetat (25 mmol/1), und Methanol, versetzt mit Ammoniumacetat (25mmol/l), mit einer Fließrate von 250 µl/Minute chromatographiert. Detektion erfolgt mit UV (245 nm) und Elektrospray (Positiv) Massenspek-trometrie an einem Quadrupol-Massenspektrometer.

Verbindung (Ia) hat ein Molekulargewicht von 418 Dalton und wird unter diesen Bedingungen als [M+NH₄]⁺ Ion bei m/z = 436 detektiert. Die Retentionszeit ist mit ca. 32,5 Minuten kürzer als die der Verbindung (IIa) mit ca. 37,5 Minuten.

### Beispiel 12

### Extraktion und präparative Reindarstellung der Verbindung (Ia) aus Schüttelkulturen der Biotransformation mit S. djakartensis NRRL B-12103

Fünfzehn 20ml-Kulturen des Stammes (S. djakartensis) in 100 ml Erlenmeyerkolben wurden nach dem in Beispiel 2 beschriebenen Verfahren angezogen und die Kulturüberstände zur Aufarbeitung von Verbindung (Ia) vereinigt. Die Aufarbeitung der vereinigten Kulturüberstände erfolgte analog Beispiel 11. Der Rückstand wurde in etwa 3 ml Methanol aufgenommen. Die Isolierung der Verbindung (Ia) erfolgte über Chromatographie an einer analytischen Reversed-Phase-HPLC-Säule (4,6 x 250 mm) mit einem Gradienten von 25 mmol/1 Ammoniumacetat in Wasser und 25mmol/l Ammoniumacetat in Methanol. Pro Lauf wurde ein Aliquot von 100 µl injiziert. UV-Detektion erfolgte bei 245 nm. Die Fraktionen wurden manuell gesammelt, vereinigt und zur Trockenen eingedampft. Die Ausbeute betrug etwa 1mg.

### Beispiel 13

### Strukturaufklärung der Verbindung (Ia)

Die präparativ isolierte Verbindung (Ia) wurde in CD₃OD aufgenommen und mit Kernresonanzspektroskopie (NMR) untersucht. Es wurden ein ¹H-NMR-, COSY-, TOCSY-, HSQC- und HMBC-Spektrum aufgenommen. Die Ergebnisse sind in der folgenden Tabelle zusammengefasst.

### NMR-Daten von Verbindung (Ia) in CD₃OD (500 MHz)

| **Position** | **δ**_{**C**} **[ppm]** | **δ**_{**H**} **[ppm]** | **Mult.** | ***J* [Hz]** | **Int.** |
|---|---|---|---|---|---|
| 1 | 174 | - | | - | - |
| 2 | 35 | 2.48 | dd | 14/3 | 1H |
| | | 3.12 | dd | 14/5 | 1H |
| 3 | 49 | 2.93 | ddd | 10/5/2 | 1H |
| 4 | 43 | 3.49 | m | | 1H |
| 5 | 129 | 5.84 | ddd | 10/3/3 | 1H |
| 6 | 131 | 5.91 | d br. | 10 | 1H |
| 7 | 42 | 2.23 | m | | 1H |
| 8 | 41 | 1.46 | m | | 1H |
| | | 1.83 | dd | 13/7 | 1H |
| 9 | 73 | 4.34 | m | | 1H |
| 10 | 40 | 1.24 | m | | 1H |
| | | 2.34 | m | | 1H |
| 11 | 47 | 0.94 | m | | 1H |
| 12 | 51 | 2.85 | m | | 1H |
| 13 | 150 | 7.00 | s br. | | 1H |
| 14 | 146 | - | | | - |
| 15 | 206 | - | | | - |
| 16 | 50 | 3.22 | m | | 1H |
| 17 | 73 | 3.49 | m | | 1H |
| 18 | 36 | 1.43 | m | | 1H |
| | | 1.48 | m | | 1H |
| 19 | 23 | 1.28 | m | | 1H |
| | | 1.71 | m | | 1H |
| 20 | 27 | 1.48 | m | | 1H |
| | | 1.63 | m | | 1H |
| 21 | 79 | 4.70 | ddd | 10/5/2 | 1H |
| 22 | 70 | 3.65 | m | | 1H |
| 23 | 18 | 1.04 | d | 7 | 3H |
| 24 | 16 | 1.15 | d | 7 | 3H |

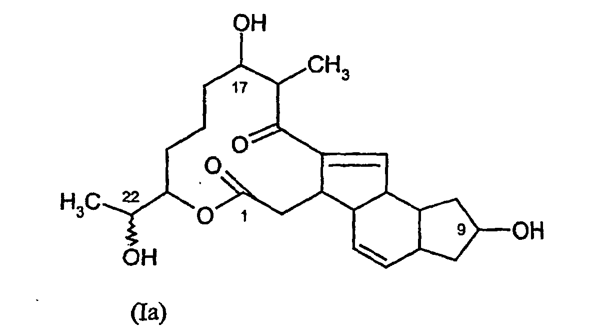

### Beispiele 14

### Analytischer Nachweis des gebildeten hydroxylierten Spinosyn A

20ml des mit Methanol versetzten Kulturüberstandes der Biotransformation aus Beispiel 8 wurden mit 0,01 N NaOH-Lösung auf pH 5 eingestellt und auf ca. 5ml wässerigen Rückstand eingeengt. Dieser wurde zweimal mit je 5ml Essigsäureethylester extrahiert. Die vereinten organischen Phasen wurden im N₂-Strom zur Trockene eingeengt und mit 200µl Methanol aufgenommen. Ein Aliquot dieses Extraktes wurde mittels LC/MS und LC/MS/MS an einem Tandem-Massenspektrometer mit Elektrospray Positiv-Ionisation untersucht.

Im LC/MS-Chromatogramm des Extraktes erscheint ein Peak bei 40,0 Minuten mit (M+H]⁺ m/z = 748,5 in geringer Konzentration. Das Tochterionenspehtrum dieses Ions weist ein Fragment mit m/z =142 auf, das charakteristisch für die Abspaltung einer Forosamin-Einheit ist.

### Beispiele 15

### Analytischer Nachweis des gebildeten hydroxylierten 17-Pseudo-spinosyn-Aglykon

20ml des mit Methanol versetzten Kulturüberstandes der Biotransformation aus Beispiel 9 wurden mit 0,01 N NaOH-Lösung auf pH 5 eingestellt und auf ca. 5ml wässerigen Rückstand eingeengt. Dieser wurde zweimal mit je 5ml Essigsäureethylester extrahiert. Die vereinten organischen Phasen wurden im N₂-Strom zur Trockene eingeengt und mit 200µl Methanol aufgenommen. Ein Aliquot dieses Extraktes wurde mittels LC/MS und LC/MS/MS an einem Tandem-Massenspektrometer mit Elektrospray Positiv-Ionisation untersucht.

Im LC/MS-Chromatogramm des Extraktes erscheint bei 38,8 Minuten ein Peaks mit [M+NH₄]⁺ m/z = 624.4 in geringer Konzentration. Dies entspricht einem hydroxylierten Produkt aus 17-Pseudo-spinosyn A-Aglykon. Das Tochterionenspektrum dieses Ions weist ein Fragment mit m/z = 189 auf, das charakteristisch für die Abspaltung einer Trimethylrhamnose-Einheit ist.

### Beispiel 16

### Charakterisierung der verwendeten Stämme

**a) Streptomyces djakartensis NRRL B-12103:**
   Dieser Stamm wurde als Niddamycin-Produzent von der Agricultural Research Service Culture Collection (1815 N. University Street, Illinois 61604, U.S.A.) unter der Zugangsnummer NRRL B-12103 bezogen. Die Stammbeschreibung kann der US 3646194 entnommen werden. Die Kultur wurde nochmals am 06.06.2001 unter der Hinterlegungsnummer DSM 14327 bei der Deutschen Sammlung von Mikroorganismen und Zellkulturen GmbH (DSMZ), Mascheroder Weg 1b, D-38124 Braunschweig, Deutschland, in Übereinstimmung mit den Bestimmungen des Budapester Vertrages hinterlegt.
**b) Streptomyces griseofuscus DSM 40191:**
   Dieser Stamm wurde als Bundlin A, B, Moldicidin A und Pentamycin-Produzent von der Deutschen Sammlung von Mikroorganismen und Zellkulturen (Mascheroder Weg 1b, D-38124 Braunschweig, Deutschland) unter der Zugangsnummer 40191 bezogen. Die Kultur wurde nochmals am 06.06.2001 unter der Hinterlegungsnummer DSM 14330 bei der Deutschen Sammlung von Mikroorganismen und Zellkulturen GmbH (DSMZ), Mascheroder Weg 1b, D-38124 Braunschweig, Deutschland, in Übereinstimmung mit den Bestimmungen des Budapester Vertrages hinterlegt.
**c) Streptomyces caelestis DSM 40084:**
   Dieser Stamm wurde als Caelesticetin-Produzent von der Deutschen Sammlung von Mikroorganismen und Zellkulturen (Mascheroder Weg 1b, D-38124 Braunschweig, Deutschland) unter der Zugangsnummer 40084 bezogen. Die Kultur wurde nochmals am 06.06.2001 unter der Hinterlegungsnummer 14328 bei der Deutschen Sammlung von Mikroorganismen und Zellkulturen GmbH (DSMZ), Mascheroder Weg 1b, D-38124 Braunschweig, Deutschland, in Übereinstimmung mit den Bestimmungen des Budapester Vertrages hinterlegt.
**d) Streptomyces antibioticus ATCC 11891:**
   Dieser Stamm wurde als Caelesticetin-Produzent von der American Type Culture Collection (10801 University Boulevard, Manassas, VA 20110-2209, USA) unter der Zugangsnummer ATCC 11891 bezogen. Die Kultur wurde nochmals am 06.06.2001 unter der Hinterlegungsnummer DSM 14329 bei der Deutschen Sammlung von Mikroorganismen und Zellkulturen GmbH (DSMZ), Mascheroder Weg 1b, D-38124 Braunschweig, Deutschland, in Übereinstimmung mit den Bestimmungen des Budapester Vertrages hinterlegt.
**e) Streptomyces griseus DSM 40937:**
   Dieser Stamm wurde von der Deutschen Sammlung von Mikroorganismen und Zellkulturen (Mascheroder Weg 1b, D-38124 Braunschweig, Deutschland) unter der Zugangsnummer 40937 bezogen. Die Kultur wurde nochmals am 06.06.2001 unter der Hinterlegungsnummer DSM 14331 bei der Deutschen Sammlung von Mikroorganismen und Zellkulturen GmbH (DSMZ), Mascheroder Weg 1b, D-38124 Braunschweig, Deutschland, in Übereinstimmung mit den Bestimmungen des Budapester Vertrages hinterlegt.
**f) Streptomyces aureofaciens DSM 46447:**
   Dieser Stamm wurde als Tetracyclin-Produzent von der Deutschen Sammlung von Mikroorganismen und Zellkulturen (Mascheroder Weg 1b, D-38124 Braunschweig, Deutschland) unter der Zugangsnummer 40084 bezogen. Die Kultur wurde nochmals am 06.06.2001 unter der Hinterlegungsnummer DSM 14332 bei der Deutschen Sammlung von Mikroorganismen und Zellkulturen GmbH (DSMZ), Mascheroder Weg 1b, D-38124 Braunschweig, Deutschland, in Übereinstimmung mit den Bestimmungen des Budapester Vertrages hinterlegt.

### Beispiel 17

### Herstellung der Ausgangsverbindungen

### Spinosyn A-Aglykon (IIa)

Das Spinosyn A-Aglykon (IIa) [Verbindung der allgemeinen Formel (II), worin R¹ für Wasserstoff, A-B für die Gruppe -HC=CH- und D für die Gruppe C-OH steht] wurde wie in WO 01/16303 beschrieben aus Tracer® hergestellt.

### 9-Keto-spinosyn A-Aglykon (IIb)

Das 9-Keto-spinosyn A-Aglykon (IIb) [Verbindung der allgemeinen Formel (II), worin R¹ für Wasserstoff, A-B für die Gruppe -HC=CH- und D für die Gruppe C=O steht] wurde mittels Pyridiniumdichromat-Oxidation aus der Verbindung (IIa) hergestellt:

46,55 g (115,6mmol) des Spinosyn A-Aglykons (IIa) wurden unter Inertgas in 1100ml abs. Dichlormethan gelöst und mit 43,51 g (115,6mmol) Pyridiniumdichromat versetzt. Nach 4 Stunden Rühren bei 25°C und Zugabe von 900 ml Diethylether wurden die ausgefallenen Chromsalze abfiltriert und das Filtrat im Vakuum eingeengt. Säulenchromatographie an Kieselgel (Eluent: Cyclohexan/Essigsäureethylester 1:1, dann 100 % Essigsäureethylester) lieferte 3,68g 9,17-Diketospinosyn-Aglykon und 23,40g einer ca. 9:1-Mischung des Spinosyn A-Aglykons (IIa) und 17-Keto-spinosyn-Aglykon (IIb) neben 11,74 g zurückgewonnenem Spinosyn A-Aglykon (IIa). Durch Umkristallisation der Mischung in Cyclohexan/Essigsäureethylester wird das 9-Keto-spinosyn A-Aglykon (IIb) auf > 98 % angereichert. Man erhält 20,78g 9-Keto-spinosyn A-Aglykon (IIb) als farblose Kristalle.

DC: *R*_{f} (SiO₂, Essigsäureethylester) = 0,44 - ¹H-NMR: CDCl₃, δ = 6,77 (s, 13-H); 5,97 (d, 6-H); 5,88 (m, 5-H); 4,72 (m, 21-H); 3,69 (m, 17-H) u. a. - LC/ESI-MS: *m*/*z* = 401 (25 %) [M]⁺, 289 (100 %).

Diketospinosyn-Aglykon: DC: *R*_{f} (SiO₂, Essigsäureethylester) = 0,64. - ¹H-NMR: CDCl₃, δ = 6,92 (s, 13-H); 5,97 (d, 6-H); 5,87 (m, 5-H); 4,85 (m, 21-H); 4,25 (q, 16-H) u. a. - LC/ESI-MS: *m*/*z* = 399 (100 %) [M+H]⁺.

### Spinosyn A

Zur Darstellung von Spinosyn A wurde zunächst wie in WO 01/16303 beschrieben verfahren. Das dabei anfallende Spinosyn A/D-Gemisch wurde durch Chromatographie an einer präparativen Reversed-Phase-Säule (250 x 8 mm) aufgetrennt. Als Eluenten wurden Wasser mit 25mmol/L Ammoniumacetat (A) und Methanol mit 25mmol/L Ammoniumacetat (B) verwendet. Die Elution erfolgte mit einem Gradienten von 60 % B auf 100 % B in 35 Minuten. Die Fließrate lag bei 3 ml/Minute. Die getrennten Substanzen wurden mit einem UV-Detektor bei 242 nm detektiert und automatisch fraktioniert. Spinosyn A eluierte bei ca. 31 Minuten und Spinosyn D bei ca. 33 Minuten. Die vereinigten Fraktionen von Spinosyn A aus mehreren Injektionen wurden an einem Rotationsverdampfer im Vakuum bis auf den wässerigen Rest eingedampft. Diese wässerige Lösung wurde einer Gefriertrocknung unterzogen und Spinosyn A als weißer Feststoff erhalten.

### 17-Pseudo-spinosyn A/D-Aglykon

Das 17-Pseudo-spinosyn A/D-Aglykon wurde wie in WO 01/16303 beschrieben, hergestellt.

## Patentansprüche

1. Verfahren zum Herstellen von Verbindungen der allgemeinen Formel (I), 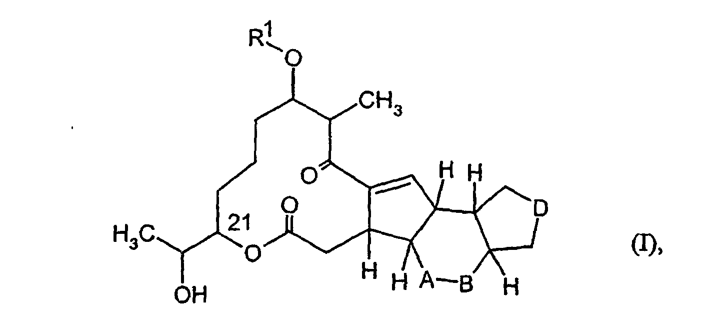 in welcher
A-B für eine der folgenden Gruppen steht: -HC=CH-, -HC=C(CH₃)-, -H₂C-CH₂- oder -H₂C-CH(CH₃)-,
D für die Gruppe 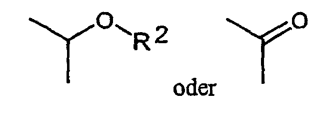 steht,
R¹ für Wasserstoff oder einen Amino-Zucker steht, und
R² für Wasserstoff oder einen Zucker steht,
**dadurch gekennzeichnet, dass** man Verbindungen der allgemeinen Formel (II), 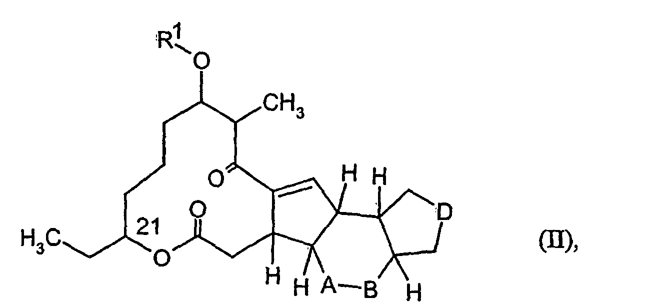 in welcher
A-B, D und R¹ die oben genannten Bedeutungen haben,
mit einem Mikroorganismus in einem wässrigen Nährmedium unter aeroben Bedingungen oder mit einem daraus hergestellten Enzymextrakt oder mit einem oder mehreren daraus isolierten Enzymen in Kontakt bringt.

2. Verfahren gemäß Anspruch 1, **dadurch gekennzeichnet, dass** Verbindungen der allgemeinen Formel (II) eingesetzt werden,
in welcher in dem Fall (1), dass
A-B für eine der folgenden Gruppen steht: -HC=CH-, -HC=C(CH₃)-, -H₂C-CH₂- oder -H₂C-CH(CH₃)-, und
D für die Gruppe 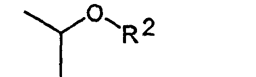 steht,
R¹ für einen Amino-Zucker der Formel 1a steht, 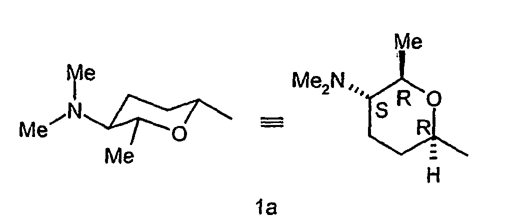 und
R² für einen Zucker der Formel 2a steht, 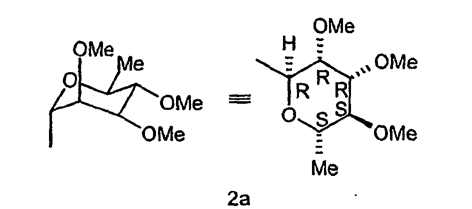
oder in welcher in dem Fall (2), dass
A-B für die Gruppe -HC=CH- oder -H₂C-CH₂- steht, und
D die oben genannte Bedeutung hat,
R¹ für einen Aminozucker der oben genannten Formel 1a steht, und
R² für Wasserstoff oder für einen Zucker der Formel 2b, 2c, 2d, 2e oder 2f steht, 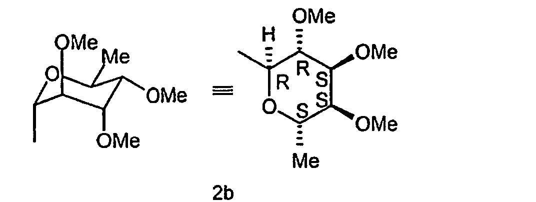 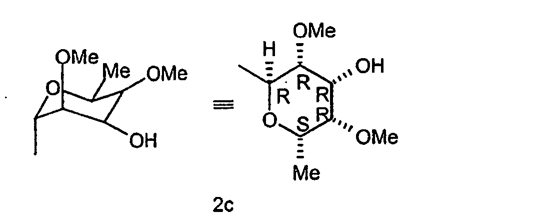 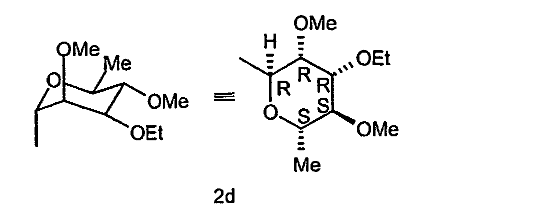 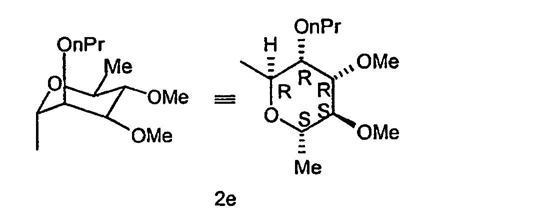 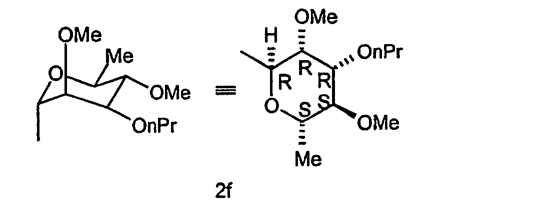
oder in welcher in dem Fall (3), dass
A-B für eine der folgenden Gruppen steht: -HC=CH-, -HC=C(CH₃)- oder -H₂C-CH₂-, und
D die oben genannte Bedeutung hat,
R¹ für Wasserstoff oder für einen Amino-Zucker der Formel 1b steht, 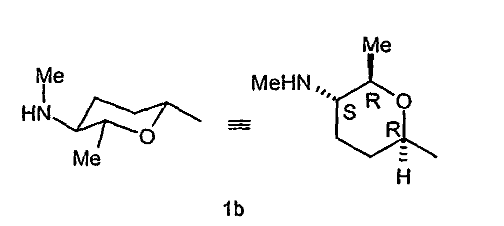
und
R² für Wasserstoff oder für einen Zucker der oben genannten Formel 2a steht,
oder in welcher in dem Fall (4), dass
A-B für die Gruppe -HC=CH- oder -HC=C(CH₃)- steht, und
D die oben genannte Bedeutung hat,
R¹ für einen Aminozucker der oben genannten Formel 1a steht, und
R² für einen Zucker der Formel 2g, 2h, 2i, 2j oder 2k steht, 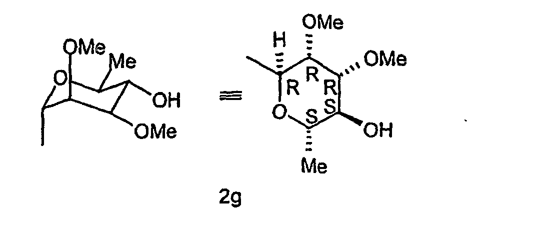 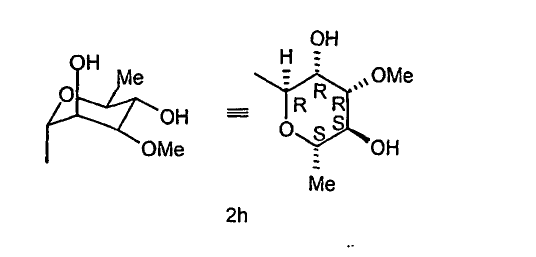 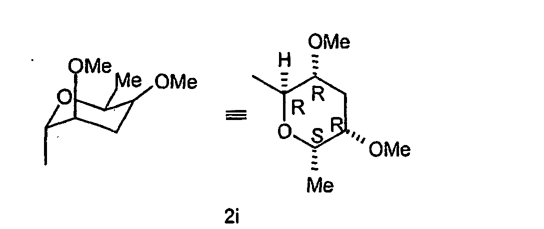 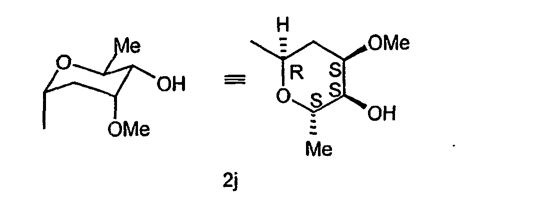 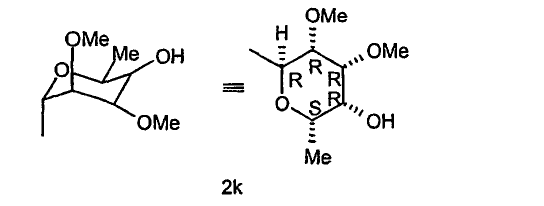
oder in welcher in dem Fall (5), dass
A-B für die Gruppe -HC=CH- oder -H₂C-CH₂- steht, und
D die oben genannte Bedeutung hat,
R¹ für einen Aminozucker der oben genannten Formel 1a steht, und
R² für einen Zucker der Formel 21 oder 2m steht, 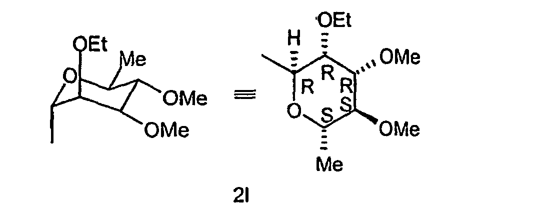 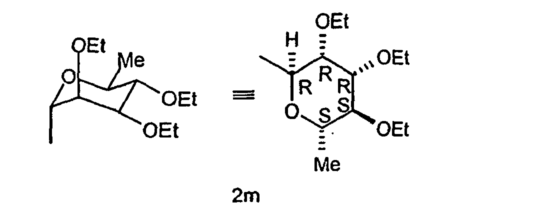
oder in welcher in dem Fall (6), dass
A-B für die Gruppe -HC=CH- oder -HC=C(CH₃)- steht, und
D die oben genannte Bedeutung hat,
R¹ für Wasserstoff oder für einen Aminozucker der oben genannten Formel 1b oder für einen Aminozucker der Formel 1c steht, 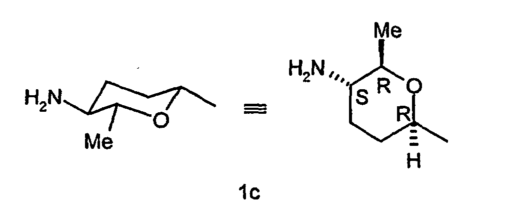
und
R² für einen Zucker der oben genannten Formel 2b, 2c, 2g oder 2h, oder für einen Zucker der Formel 2n steht, 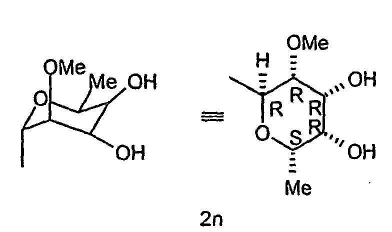
oder in welcher in dem Fall (7), dass
A-B für die Gruppe -HC=CH- steht, und
D die oben genannte Bedeutung hat,
R¹ für einen Aminozucker der oben genannten Formel 1a steht, und
R² für einen Zucker der Formel 2o steht, 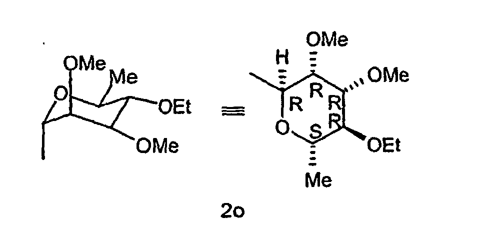
oder in welcher in dem Fall (8), dass
A-B für die Gruppe -HC=CH- steht, und
D die oben genannte Bedeutung hat,
R¹ für einen Aminozucker der oben genannten Fonnel 16 steht, und
R² für einen Zucker der oben genannten Formel 2d, 2i oder 2j, oder für einen Zucker der Fomiel 2p steht, 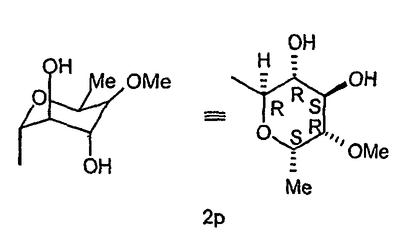
oder in welcher in dem Fall (9), dass
A-B für die Gruppe -HC=CH- steht, und
D die oben genannte Bedeutung hat,
R¹ für Wasserstoff oder für einen Aminozucker der oben genannten Formel 1c steht, und
R² für einen Zucker der oben genannten Formel 2i oder 2p steht,
oder in welcher in dem Fall (10), dass
A-B für die Gruppe -HC=CH- steht, und
D die oben genannte Bedeutung hat,
R¹ für einen Aminozucker der Formel 1d, 1e oder 1f steht, 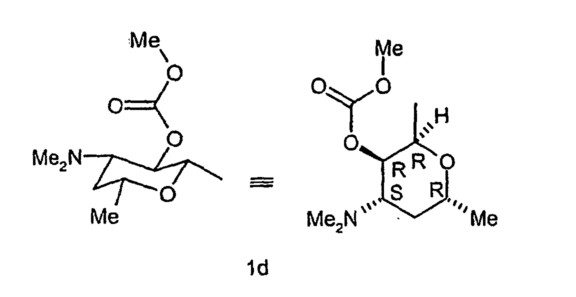 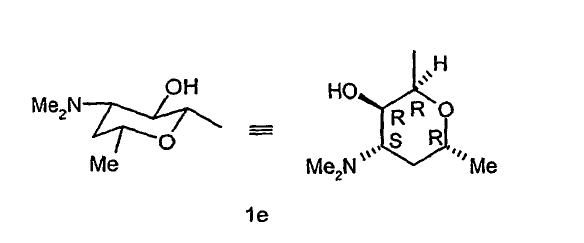 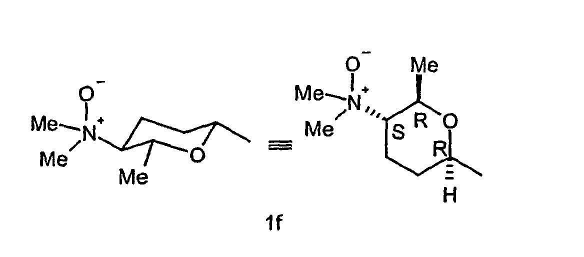
und
R² für einen Zucker der oben genannten Fonnel 2a steht,
oder in welcher in dem Fall (11), dass
A-B für die Gruppe -HC=CH- steht, und
D für die Gruppe 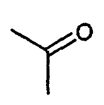 steht,
R¹ für Wasserstoff oder für einen Aminozucker der oben genannten Formel 1a steht.

3. Verfahren gemäß Anspruch 1 oder 2, **dadurch gekennzeichnet, dass** Verbindungen der allgemeinen Formel (II) eingesetzt werden,
in welcher in dem Fall (12), dass
A-B für die Gruppe -HC=CH- oder -HC=C(CH₃)- steht, und
D für die Gruppe 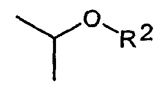 steht,
R¹ für einen Amino-Zucker der Formel 1a steht, und
R² für einen Zucker der Formel 2a, 2g oder 2h steht,
oder in welcher in dem Fall (13), dass
A-B für die Gruppe -HC=CH- steht, und
D die oben genannte Bedeutung hat,
R¹ für einen Aminozucker der Formel 1a steht, und
R² für Wasserstoff oder für einen Zucker der Formel 2d, 2e, 21, 2m oder 2o steht,
oder in welcher in dem Fall (14), dass
A-B für die Gruppe -HC=CH- oder -HC=C(CH₃)- steht, und
D die oben genannte Bedeutung hat,
R¹ für Wasserstoff oder für einen Amino-Zucker der Formel 1b steht, und
R² für Wasserstoff oder für einen Zucker der Formel 2a steht,
oder in welcher A-B, D und R¹ wie im Fall (11) definiert sind.

4. Verfahren gemäß einem der Ansprüche 1 bis 3, **dadurch gekennzeichnet, dass** Verbindungen der allgemeinen Formel (II) eingesetzt werden,
in welcher in dem Fall (15), dass
A-B für die Gruppe -HC=CH- oder -HC=C(CH₃)- steht, und
D für die Gruppe 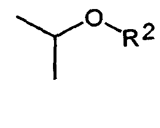 steht,
R¹ für einen Amino-Zucker der Formel 1a steht, und
R² für einen Zucker der Formel 2a steht,
oder in welcher in dem Fall (16), dass
A-B für die Gruppe -HC=CH- steht, und
D die oben genannte Bedeutung hat,
R¹ für einen Aminozucker der Formel 1a steht, und
R² für Wasserstoff oder für einen Zucker der Formel 2d, 21 oder 2m steht,
oder in welcher A-B, D und R¹ wie im Fall (11) definiert sind.

5. Verfahren gemäß einem der Ansprüche 1 bis 4, **dadurch gekennzeichnet, dass** Verbindungen der allgemeinen Formel (II) eingesetzt werden,
in welcher in dem Fall (17), dass
A-B für die Gruppe -HC=CH- oder -HC=C(CH₃)- steht, und
D für die Gruppe 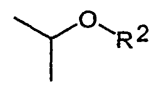 steht,
R¹ für einen Amino-Zucker der Formel 1a steht, und
R² für einen Zucker der Formel 2a steht,
oder in welcher in dem Fall (18), dass
A-B für die Gruppe -HC=CH- steht, und
D die oben genannte Bedeutung hat,
R¹ für Wasserstoff steht, und
R² für Wasserstoff steht.

6. Verfahren gemäß einem der Ansprüche 1 bis 5, **dadurch gekennzeichnet, dass** man als Mikroorganismus einen Mikroorganismus aus der Gruppe der Actinomyceten einsetzt.

7. Verfahren gemäß Anspruch 6, **dadurch gekennzeichnet, dass** man als Mikroorganismus aus der Gruppe der Actinomyceten einen Mikroorganismus aus der Gattung Streptomyces einsetzt.

8. Verfahren gemäß Anspruch 7, **dadurch gekennzeichnet, dass** man als Mikroorganismus aus der Gattung Streptomyces einen Stamm aus der Art Streptomyces djakartensis, Streptomyces griseofuscus, Streptomyces caelestis, Streptomyces antibioticus, Streptomyces griseus oder Streptomyces aureofaciens einsetzt.

9. Verfahren gemäß Anspruch 8, **dadurch gekennzeichnet, dass** der Stamm die kennzeichnenden Merkmale der Stämme Streptomyces djakartensis DSM 14327, Streptomyces griseofuscus DSM 14330, Streptomyces caelestis DSM 14328, Streptomyces antibioticus DSM 14329, Streptomyces griseus DSM 14331 oder Streptomyces aureofaciens DSM 14332 aufweist.

10. Verfahren gemäß einem der Ansprüche 1 bis 9, **dadurch gekennzeichnet, dass** man die Verbindungen der allgemeinen Formel (I) isoliert.

11. Verbindungen der allgemeinen Formel (I), 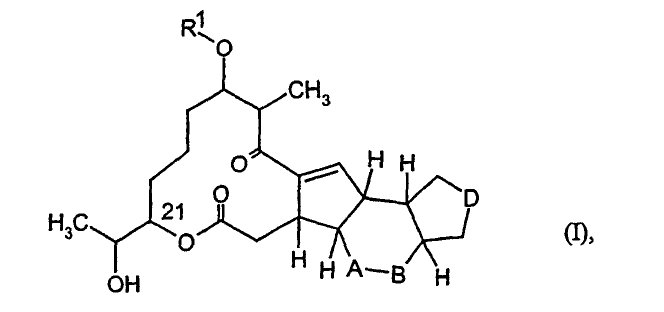 in welcher
A-B für eine der folgenden Gruppen steht: -HC=CH-, -HC=C(CH₃)-, -H₂C-CH₂- oder -H₂C-CH(CH₃)-,
D für die Gruppe 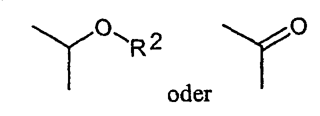 steht,
R¹ für einen Amino-Zucker steht, und
R² für einen Zucker steht.

12. Verbindungen gemäß Anspruch 11, **dadurch gekennzeichnet, dass**
in dem Fall ( 1 ), dass
A-B für eine der folgenden Gruppen steht: -HC=CH-, -HC=C(CH₃)-, -H₂C-CH₂- oder -H₂C-CH(CH₃)-, und
D für die Gruppe 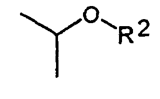 steht,
R¹ für einen Amino-Zucker der Formel 1a steht, 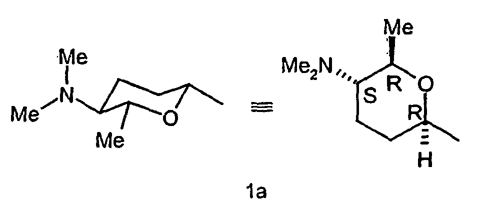 und
R² für einen Zucker der Formel 2a steht, 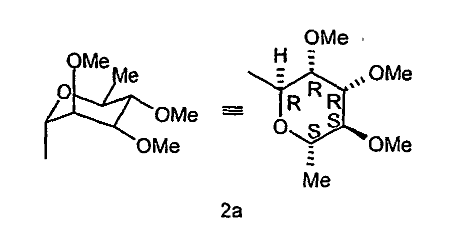
oder in dem Fall (2), dass
A-B für die Gruppe -HC=CH- oder -H₂C-CH₂- steht, und
D die oben genannte Bedeutung hat,
R¹ für einen Aminozucker der oben genannten Formel 1a steht, und
R² für Wasserstoff oder für einen Zucker der Formel 2b, 2c, 2d, 2e oder 2f steht, 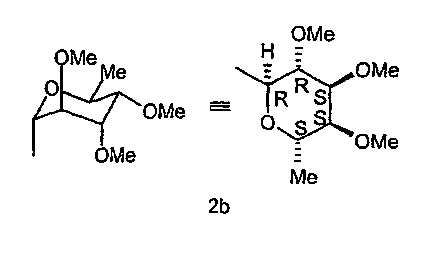 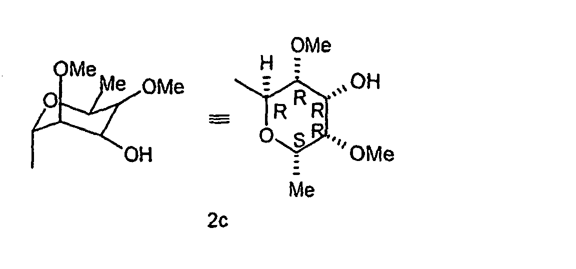 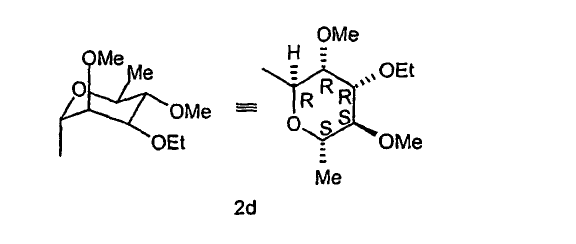 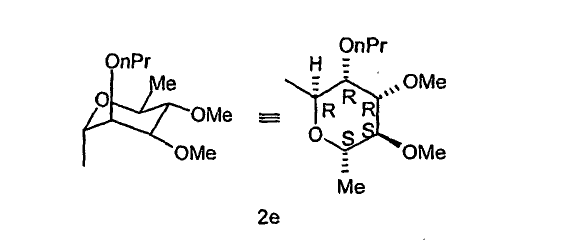 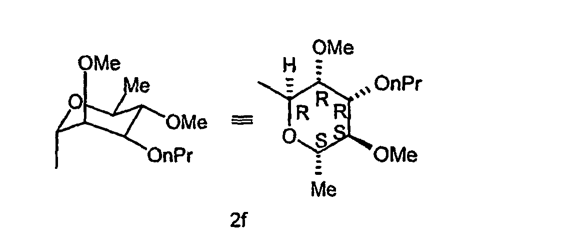
oder in dem Fall (3), dass
A-B für eine der folgenden Gruppen steht: -HC=CH-, -HC=C(CH₃)- oder -H₂C-CH₂-, und
D die oben genannte Bedeutung hat,
R¹ für Wasserstoff oder für einen Amino-Zucker der Formel 1b steht, 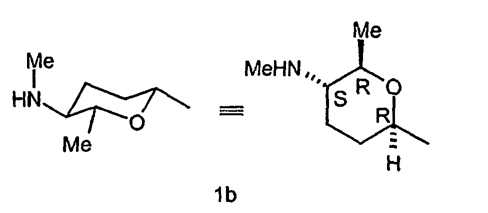 und
R² für Wasserstoff oder für einen Zucker der oben genannten Formel 2a steht,
oder in dem Fall (4), dass
A-B für die Gruppe -HC=CH- oder -HC=C(CH₃)- steht, und
D die oben genannte Bedeutung hat,
R¹ für einen Aminozucker der oben genannten Formel 1a steht, und
R² für einen Zucker der Formel 2g, 2h, 2i, 2j oder 2k steht, 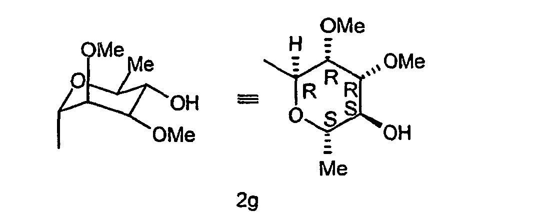 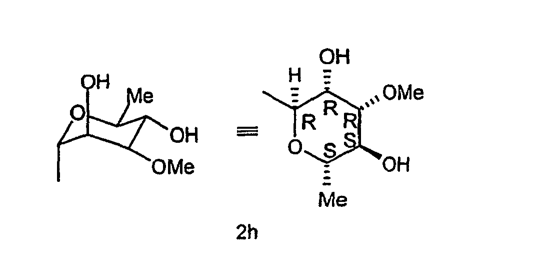 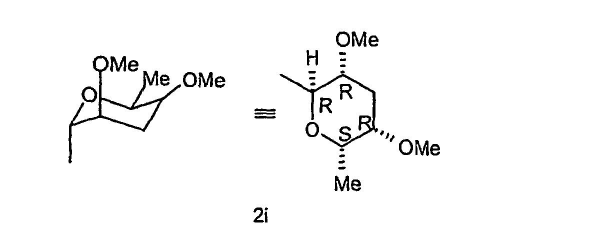 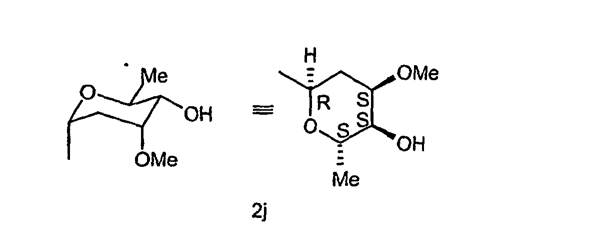 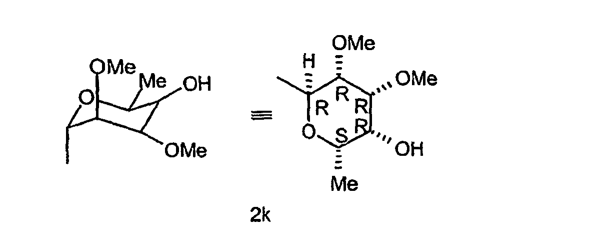
oder in dem Fall (5), dass
A-B für die Gruppe -HC=CH- oder -H₂C-CH₂- steht, und
D die oben genannte Bedeutung hat,
R¹ für einen Aminozucker der oben genannten Formel 1a steht, und
R² für einen Zucker der Formel 21 oder 2m steht, 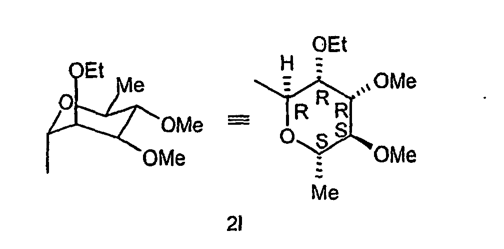 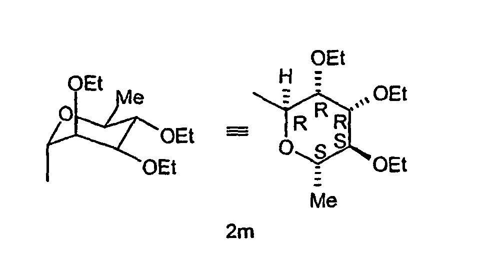
oder in dem Fall (6), dass
A-B für die Gruppe -HC=CH- oder -HC=C(CH₃)- steht, und
D die oben genannte Bedeutung hat,
R¹ für Wasserstoff oder für einen Aminozucker der oben genannten Formel 1b oder für einen Aminozucker der Formel 1c steht, 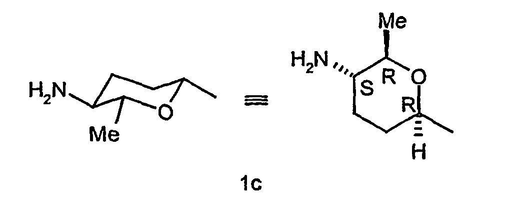 und
R² für einen Zucker der oben genannten Formel 2b, 2c, 2g oder 2h, oder für einen Zucker der Formel 2n steht, 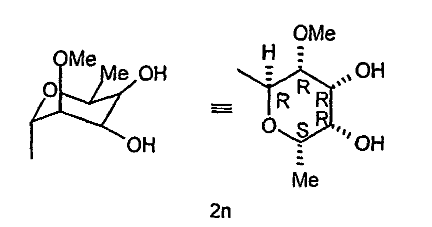
oder in dem Fall (7), dass
A-B für die Gruppe -HC=CH- steht, und
D die oben genannte Bedeutung hat,
R¹ für einen Aminozucker der oben genannten Formel 1a steht, und
R² für einen Zucker der Formel 2o steht, 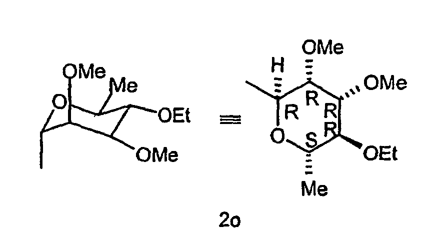
oder in dem Fall (8), dass
A-B für die Gruppe -HC=CH- steht, und
D die oben genannte Bedeutung hat,
R¹ für einen Aminozucker der oben genannten Formel 1b steht, und
R² für einen Zucker der oben genannten Formel 2d, 2i oder 2j, oder für einen Zucker der Formel 2p steht, 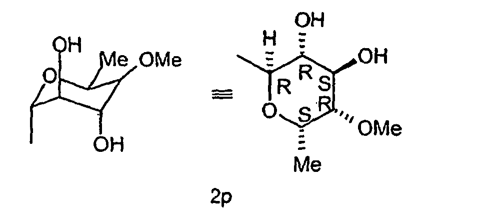
oder in dem Fall (9), dass
A-B für die Gruppe -HC=CH- steht, und
D die oben genannte Bedeutung hat,
R¹ für Wasserstoff oder für einen Aminozucker der oben genannten Formel 1c steht, und
R² für einen Zucker der oben genannten Formel 2i oder 2p steht,
oder in dem Fall (10), dass
A-B für die Gruppe -HC=CH- steht, und
D die oben genannte Bedeutung hat,
R¹ für einen Aminozucker der oben genannten Formel 1d, 1e oder für einen Aminozucker der Formel 1f steht, 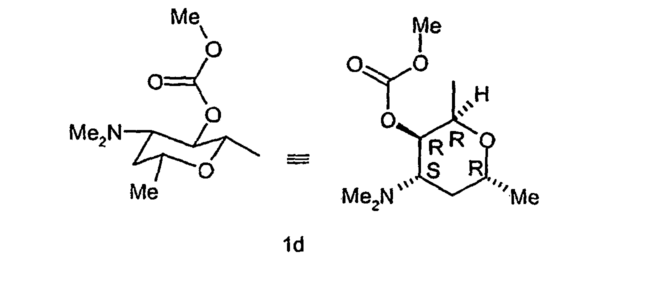 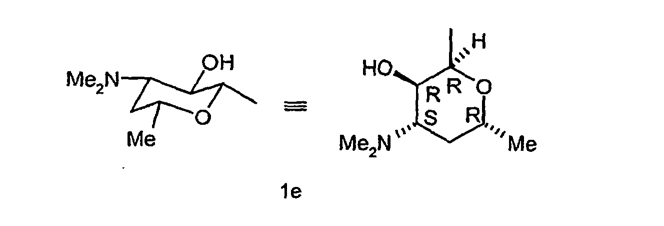 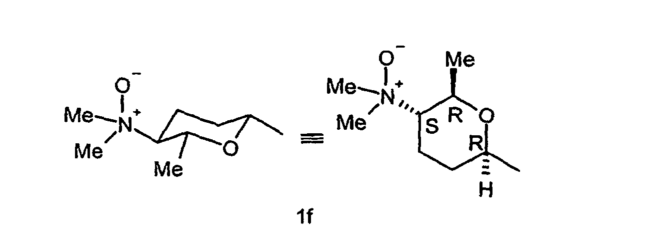
und
R² für einen Zucker der oben genannten Formel 2a steht,
oder in dem Fall (11), dass
A-B für die Gruppe -HC=CH- steht, und
D für die Gruppe 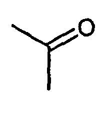 steht,
R¹ für Wasserstoff oder für einen Aminozucker der oben genannten Formel 1a steht.

13. Verbindungen gemäß Anspruch 12, **dadurch gekennzeichnet, dass**
in dem Fall (12), dass
A-B für die Gruppe -HC=CH- oder -HC=C(CH₃)- steht, und
D für die Gruppe 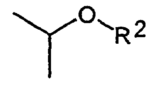 steht,
R¹ für einen Amino-Zucker der Formel 1a steht, und
R² für einen Zucker der Formel 2a, 2g oder 2h steht,
oder in dem Fall (13), dass
A-B für die Gruppe -HC=CH- steht, und
D die oben genannte Bedeutung hat,
R¹ für einen Aminozucker der Formel 1a steht, und
R² für Wasserstoff oder für einen Zucker der Formel 2d, 2e, 2l, 2m oder 2o steht,
oder in dem Fall (14), dass
A-B für die Gruppe -HC=CH- oder -HC=C(CH₃)- steht, und
D die oben genannte Bedeutung hat,
R¹ für Wasserstoff oder für einen Amino-Zucker der Formel 1b steht, und
R² für Wasserstoff oder für einen Zucker der Formel 2a steht,
oder A-B, D und R¹ wie im Fall (11) definiert sind.

14. Verbindungen gemäß Anspruch 12, **dadurch gekennzeichnet, dass**
in dem Fall (15), dass
A-B für die Gruppe -HC=CH- oder -HC=C(CH₃)- steht, und
D für die Gruppe 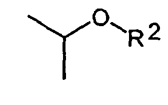 steht,
R¹ für einen Amino-Zucker der Formel 1a steht, und
R² für einen Zucker der Formel 2a steht,
oder in dem Fall (16), dass
A-B für die Gruppe -HC=CH- steht, und
D die oben genannte Bedeutung hat,
R¹ für einen Aminozucker der Formel 1a steht, und
R² für Wasserstoff oder für einen Zucker der Formel 2d, 2l oder 2m steht,
oder A-B, D und R¹ wie im Fall (11) definiert sind.

15. Verbindungen gemäß Anspruch 12, **dadurch gekennzeichnet, dass**
in dem Fall (17), dass
A-B für die Gruppe -HC=CH- oder -HC=C(CH₃)- steht, und
D für die Gruppe 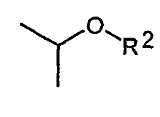 steht,
R¹ für einen Amino-Zucker der Formel 1a steht, und
R² für einen Zucker der Formel 2a steht,
oder in dem Fall (18), dass
A-B für die Gruppe -HC=CH- steht, und
D die oben genannte Bedeutung hat,
R¹ für Wasserstoff steht, und
R² für Wasserstoff steht.

16. Verbindungen gemäß Anspruch 12, **dadurch gekennzeichnet, dass**
A-B für die Gruppe -HC=CH- steht,
D für die Gruppe 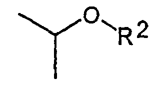 steht,
R¹ für einen Amino-Zucker der Formel 1a steht, und
R² für einen Zucker der Formel 2a steht,
oder in welcher
A-B für die Gruppe -HC=CH- oder -HC=C(CH₃)- steht,
D die oben genannte Bedeutung hat,
R¹ für Wasserstoff steht, und
R² für einen Zucker der Formel 2a steht,
oder in welcher
A-B für eine Gruppe -HC=CH- steht,
D die oben genannte Bedeutung hat,
R¹ für Wasserstoff steht, und
R² für Wasserstoff steht.

17. Verwendung von Verbindungen gemäß einem der Ansprüche 11 bis 16 zum Herstellen von neuen Spinosyn-Derivaten.

## Claims

1. Method for producing compounds of the general formula (I), 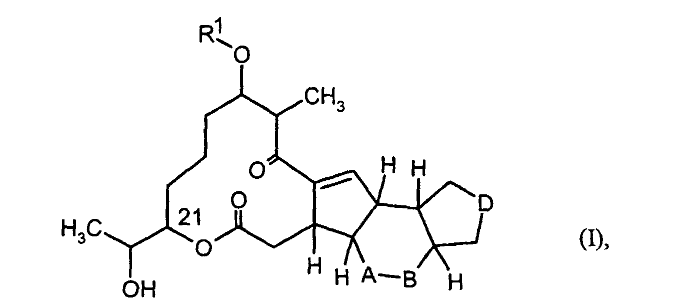 in which
A-B is any of the following groups: -HC=CH-, -HC=C(CH₃)-, -H₂C-CH₂- or -H₂C-CH(CH₃)-,
D is the group 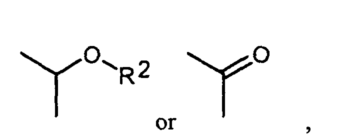
R¹ is hydrogen or an amino sugar and
R² is hydrogen or a sugar,
**characterized in that** compounds of the general formula (II), 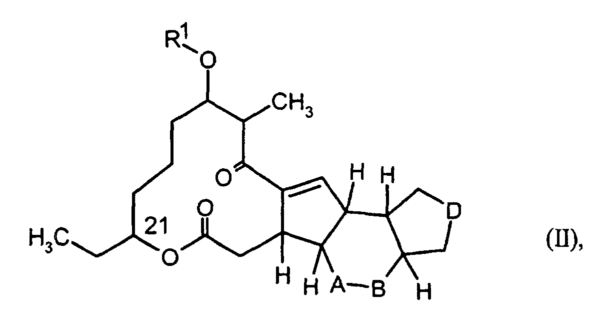 in which
A-B, D and R¹ are as defined above,
are contacted with a microorganism in an aqueous nutrient medium under aerobic conditions or with an enzyme extract prepared therefrom or with one or more enzymes isolated therefrom.

2. Method according to Claim 1, **characterized in that** compounds of the general formula (II) are used,
in which formula, in the case (1) that
A-B is any of the following groups: -HC=CH-, -HC=C(CH₃)-, -H₂C-CH₂- or -H₂C-CH(CH₃)- and
D is the group 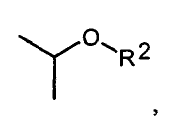
R¹ is an amino sugar of the formula 1a 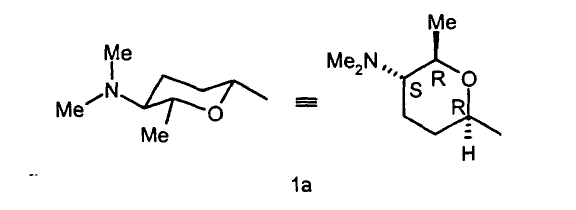 and
R² is a sugar of the formula 2a 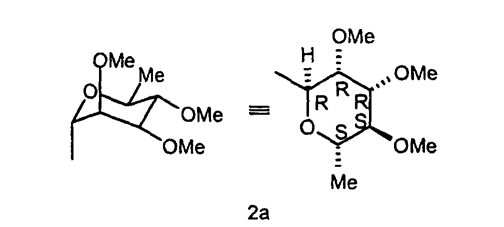
or in which, in the case (2) that
A-B is the group -HC=CH- or -H₂C-CH₂- and
D is as defined above,
R' is an amino sugar of the abovementioned formula 1a and
R² is hydrogen or a sugar of the formula 2b, 2c, 2d, 2e or 2f 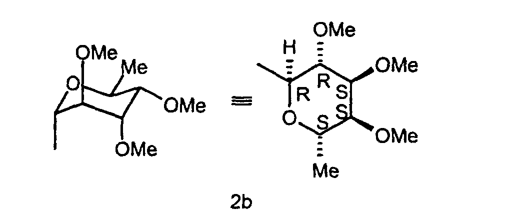 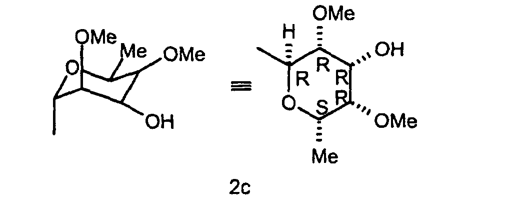 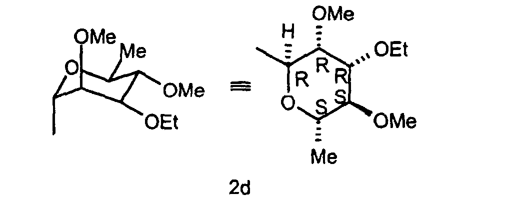 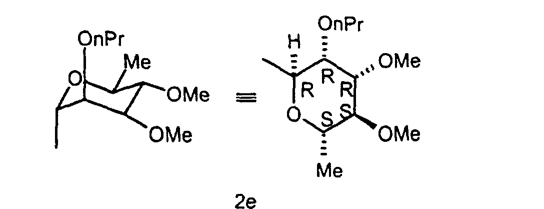 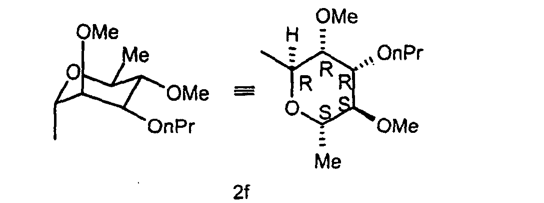
or in which, in the case (3) that
A-B is any of the following groups: -HC=CH-, -HC=C(CH₃)- or -H₂C-CH₂- and
D is as defined above,
R¹ is hydrogen or an amino sugar of the formula 1b 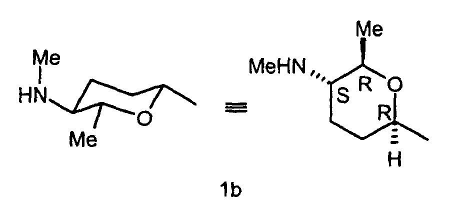
and
R² is hydrogen or a sugar of the abovementioned formula 2a
or in which, in the case (4) that
A-B is the group -HC=CH- or -HC=C(CH₃)- and
D is as defined above,
R¹ is an amino sugar of the abovementioned formula 1a and
R² is a sugar of the formula 2g, 2h, 2i, 2j or 2k 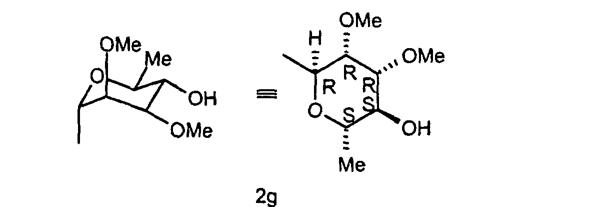 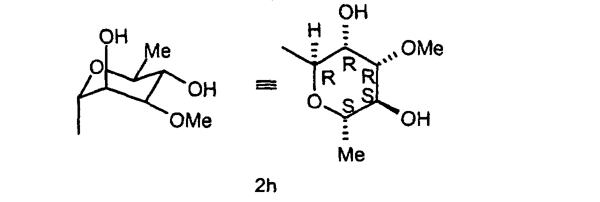 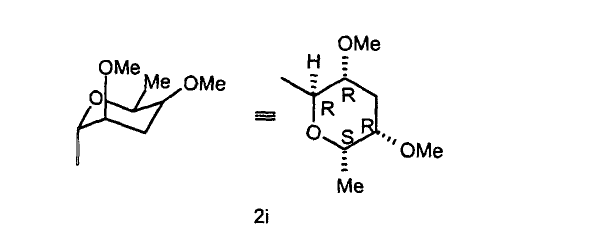 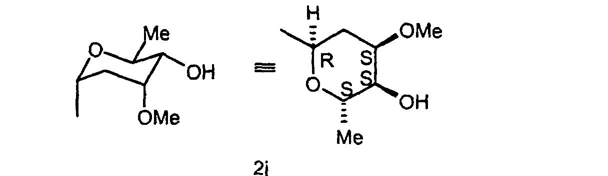 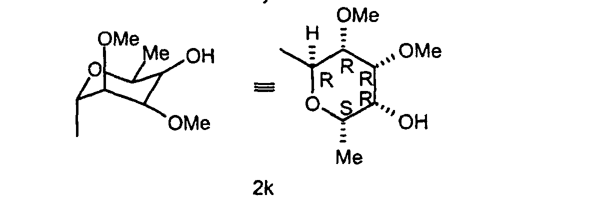
or in which, in the case (5) that
A-B is the group -HC=CH- or -H₂C-CH₂- and
D is as defined above,
R¹ is an amino sugar of the abovementioned formula 1a and
R² is a sugar of the formula 21 or 2m 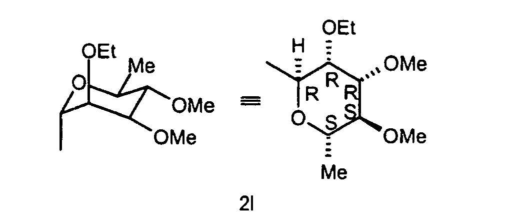 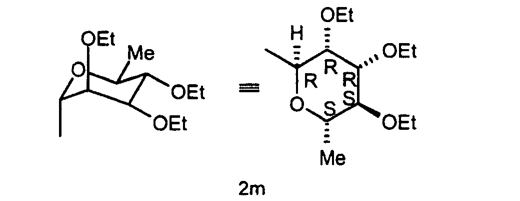
or in which, in the case (6) that
A-B is the group -HC=CH- or -HC=C(CH₃)- and
D is as defined above,
R¹ is hydrogen or an amino sugar of the abovementioned formula 1b or an amino sugar of the formula 1c 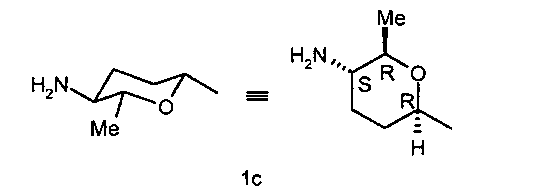
and
R² is a sugar of the abovementioned formula 2b, 2c, 2g or 2h or a sugar of the formula 2n 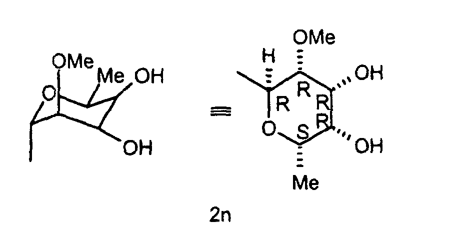
or in which, in the case (7) that
A-B is the group -HC=CH- and
D is as defined above,
R¹ is an amino sugar of the abovementioned formula 1a and
R² is a sugar of the formula 2o 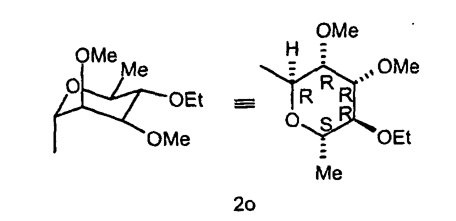
or in which, in the case (8) that
A-B is the group -HC=CH- and
D is as defined above,
R¹ is an amino sugar of the abovementioned formula 1b and
R² is a sugar of the abovementioned formula 2d, 2i or 2j or a sugar of the formula 2p 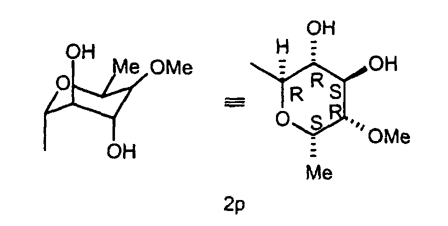
or in which, in the case (9) that
A-B is the group -HC=CH- and
D is as defined above,
R¹ is hydrogen or an amino sugar of the abovementioned formula 1c and
R² is a sugar of the abovementioned formula 2i or 2p
or in which, in the case (10) that
A-B is the group -HC=CH- and
D is as defined above,
R¹ is an amino sugar of the formula 1d, 1e or 1f 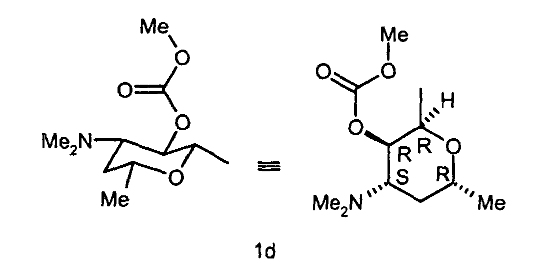 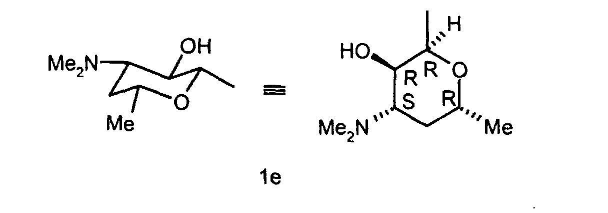 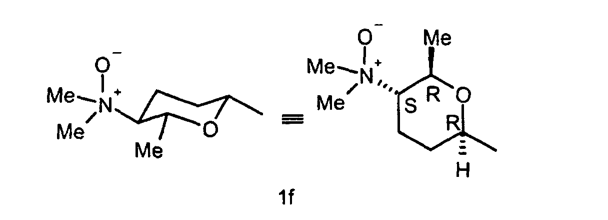
and
R² is a sugar of the abovementioned formula 2a
or in which, in the case (11) that
A-B is the group -HC=CH- and
D is the group 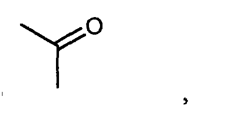
R¹ is hydrogen or an amino sugar of the abovementioned formula 1a.

3. Method according to Claim 1 or 2, **characterized in that** compounds of the general formula (II) are used,
in which formula, in the case (12) that
A-B is the group -HC=CH- or -HC=C(CH₃)- and
D is the group 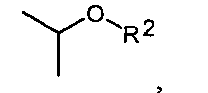
R¹ is an amino sugar of the formula 1a and
R² is a sugar of the formula 2a, 2g or 2h
or in which, in the case (13) that
A-B is the group -HC=CH- and
D is as defined above,
R¹ is an amino sugar of the formula 1a and
R² is hydrogen or a sugar of the formula 2d, 2e, 2l, 2m or 2o
or in which, in the case (14) that
A-B is the group -HC=CH- or -HC=C(CH₃)- and
D is as defined above,
R¹ is hydrogen or an amino sugar of the formula 1b and
R² is hydrogen or a sugar of the formula 2a
or in which A-B, D and R¹ are as defined as in the case (11).

4. Method according to any of Claims 1 to 3, **characterized in that** compounds of the general formula (II) are used,
in which formula, in the case (15) that
A-B is the group -HC=CH- or -HC=C(CH₃)- and
D is the group 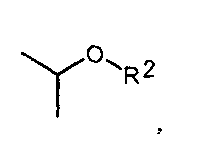
R¹ is an amino sugar of the formula 1a and
R² is a sugar of the formula 2a
or in which, in the case (16) that
A-B is the group -HC=CH- and
D is as defined above,
R¹ is an amino sugar of the formula 1a and
R² is hydrogen or a sugar of the formula 2d, 2l or 2m
or in which A-B, D and R¹ are as defined in the case (11).

5. Method according to any of Claims 1 to 4, **characterized in that** compounds of the general formula (II) are used,
in which formula, in the case (17) that
A-B is the group -HC=CH- or -HC=C(CH₃)- and
D is the group 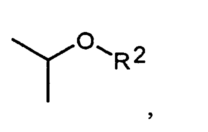
R¹ is an amino sugar of the formula 1a and
R² is a sugar of the formula 2a
or in which, in the case (18) that
A-B is the group -HC=CH- and
D is as defined above,
R¹ is hydrogen and
R² is hydrogen.

6. Method according to any of Claims 1 to 5, **characterized in that** the microorganism used is a microorganism from the group of actinomycetes.

7. Method according to Claim 6, **characterized in that** the microorganism used from the group of actinomycetes is a microorganism of the genus Streptomyces.

8. Method according to Claim 7, **characterized in that** the microorganism used of the genus Streptomyces is a strain of the species Streptomyces djakartensis, Streptomyces griseofuscus, Streptomyces caelestis, Streptomyces antibioticus, Streptomyces griseus or Streptomyces aureofaciens.

9. Method according to Claim 8, **characterized in that** the strain has the characteristic features of the strains Streptomyces djakartensis DSM 14327, Streptomyces griseofuscus DSM 14330, Streptomyces caelestis DSM 14328, Streptomyces antibioticus DSM 14329, Streptomyces griseus DSM 14331 or Streptomyces aureofaciens DSM 14332.

10. Method according to any of Claims 1 to 9, **characterized in that** the compounds of the general formula (I) are isolated.

11. Compound of the general formula (I), 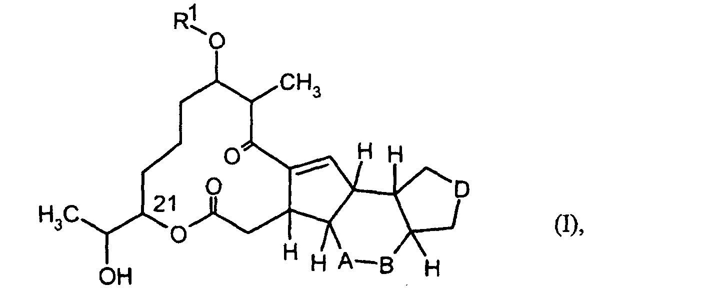 in which
A-B is any of the following groups: -HC=CH-, -HC=C(CH₃)-, -H₂C-CH₂- or -H₂C-CH(CH₃)-,
D is the group 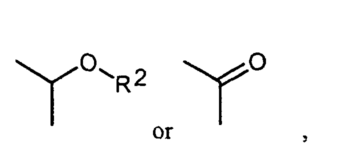
R¹ is an amino sugar and
R² is a sugar.

12. Compound according to Claim 11, **characterized in that**,
in the case (1) that
A-B is any of the following groups: -HC=CH-, -HC=C(CH₃)-, -H₂C-CH₂- or -H₂C-CH(CH₃)- and
D is the group 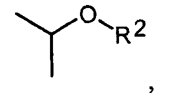
R¹ is an amino sugar of the formula 1a 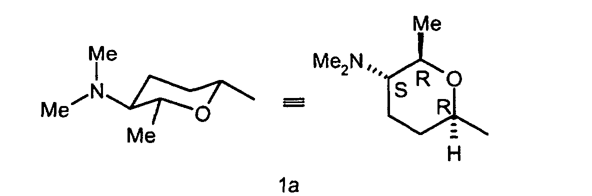 and
R² is a sugar of the formula 2a 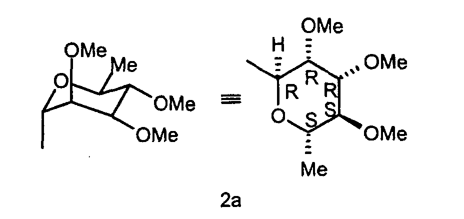
or, in the case (2) that
A-B is the group -HC=CH- or -H₂C-CH₂- and
D is as defined above,
R¹ is an amino sugar of the abovementioned formula 1a and
R² is hydrogen or a sugar of the formula 2b, 2c, 2d, 2e or 2f 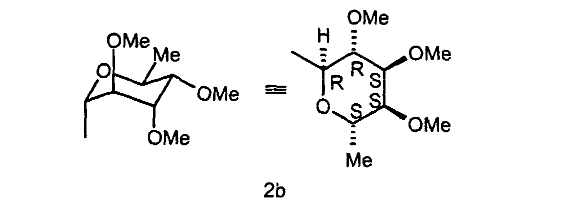 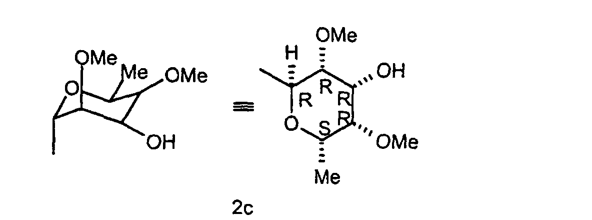 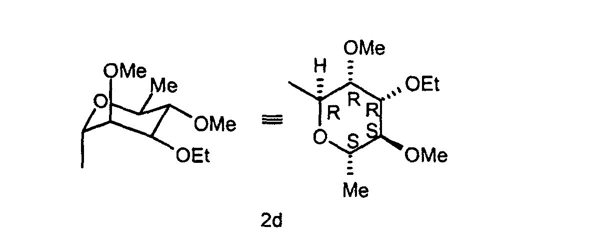 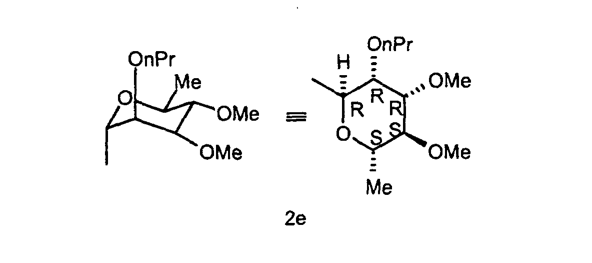 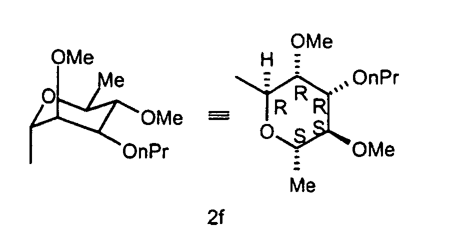
or, in the case (3) that
A-B is one of the following groups: -HC=CH-, -HC=C(CH₃)- or -H₂C-CH₂- and
D is as defined above,
R¹ is hydrogen or an amino sugar of the formula 1b 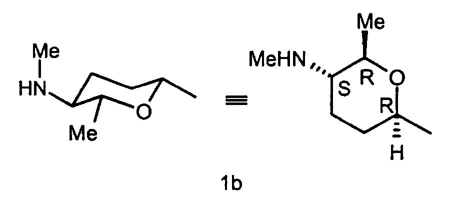 and
R² is hydrogen or a sugar of the abovementioned formula 2a
or, in the case (4) that
A-B is the group -HC=CH- or -HC=C(CH₃)- and
D is as defined above,
R¹ is an amino sugar of the abovementioned formula 1a and
R² is a sugar of the formula 2g, 2h, 2i, 2j or 2k 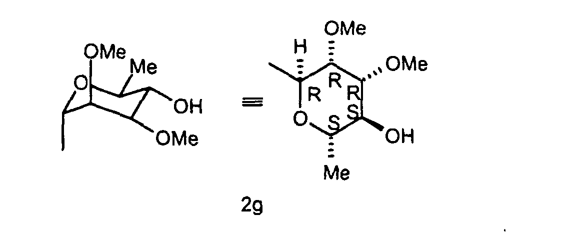 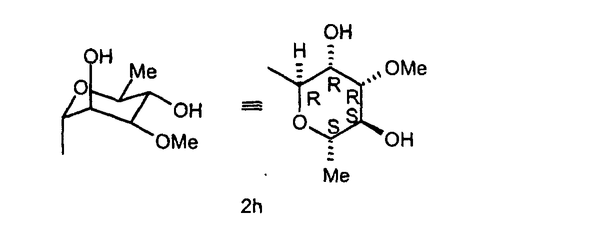 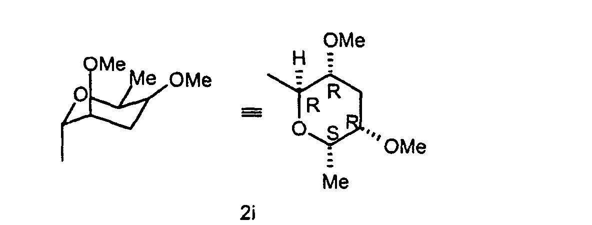 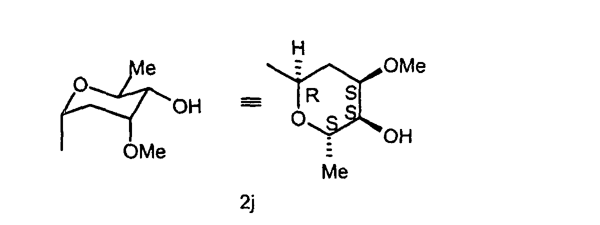 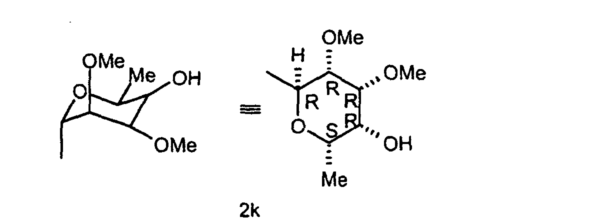
or, in the case (5) that
A-B is the group -HC=CH- or -H₂C-CH₂- and
D is as defined above,
R¹ is an amino sugar of the abovementioned formula 1a and
R² is a sugar of the formula 2l or 2m 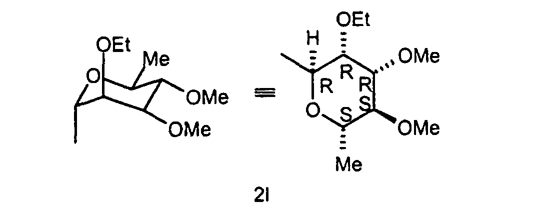 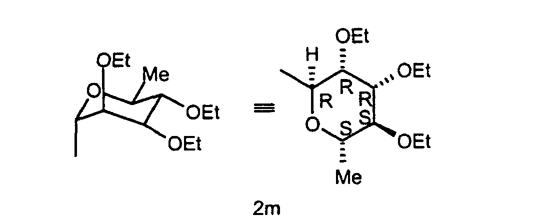
or, in the case (6) that
A-B is the group -HC=CH- or -HC=C(CH₃)- and
D is as defined above,
R¹ is hydrogen or an amino sugar of the abovementioned formula 1b or an amino sugar of the formula 1 c 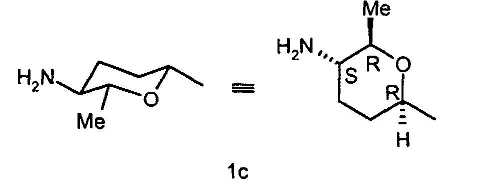 and
R² is a sugar of the abovementioned formula 2b, 2c, 2g or 2h or a sugar of the formula 2n 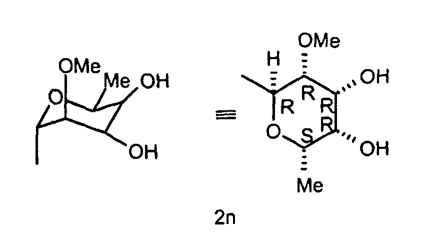
or, in the case (7) that
A-B is the group -HC=CH- and
D is as defined above,
R¹ is an amino sugar of the abovementioned formula 1a and
R² is a sugar of the formula 2o 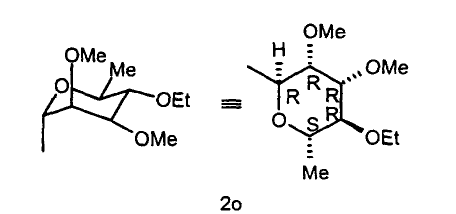
or, in the case (8) that
A-B is the group -HC=CH- and
D is as defined above,
R¹ is an amino sugar of the abovementioned formula 1b and
R² is a sugar of the abovementioned formula 2d, 2i or 2j or a sugar of the formula 2p 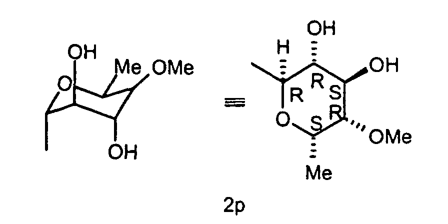
or, in the case (9) that
A-B is the group -HC=CH- and
D is as defined above,
R¹ is hydrogen or an amino sugar of the abovementioned formula 1c and
R² is a sugar of the abovementioned formula 2i or 2p
or, in the case (10) that
A-B is the group -HC=CH- and
D is as defined above,
R¹ is an amino sugar of the abovementioned formula 1d, 1e or an amino sugar of the formula 1f 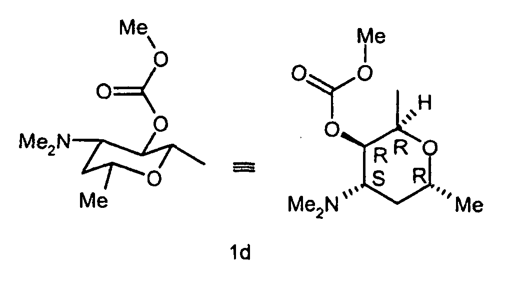 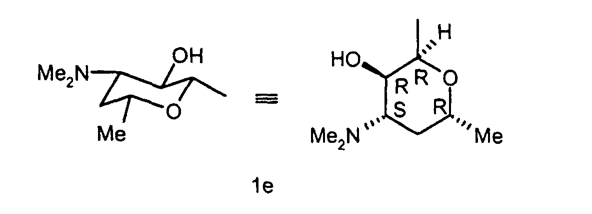 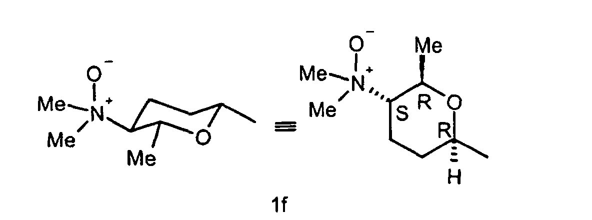
and
R² is a sugar of the abovementioned formula 2a
or, in the case (11) that
A-B is the group -HC=CH- and
D is the group 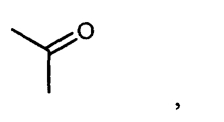
R¹ is hydrogen or an amino sugar of the abovementioned formula 1a.

13. Compound according to Claim 12, **characterized in that**,
in the case (12) that
A-B is the group -HC=CH- or -HC=C(CH₃)- and
D is the group 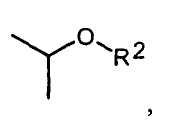
R¹ is an amino sugar of the formula 1a and
R² is a sugar of the formula 2a, 2g or 2h
or, in the case (13) that
A-B is the group -HC=CH- and
D is as defined above,
R¹ is an amino sugar of the formula 1a and
R² is hydrogen or a sugar of the formula 2d, 2e, 2l, 2m or 2o
or, in the case (14) that
A-B is the group -HC=CH- or -HC=C(CH₃)- and
D is as defined above,
R¹ is hydrogen or an amino sugar of the formula 1b and
R² is hydrogen or a sugar of the formula 2a
or A-B, D and R¹ are as defined as in the case (11).

14. Compound according to Claim 12, **characterized in that**,
in the case (15) that
A-B is the group -HC=CH- or -HC=C(CH₃)- and
D is the group 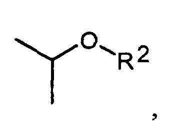
R¹ is an amino sugar of the formula 1a and
R² is a sugar of the formula 2a
or, in the case (16) that
A-B is the group -HC=CH- and
D is as defined above,
R¹ is an amino sugar of the formula 1a and
R² is hydrogen or a sugar of the formula 2d, 2l or 2m
or A-B, D and R¹ are as defined as in the case (11).

15. Compound according to Claim 12, **characterized in that**,
in the case (17) that
A-B is the group -HC=CH- or -HC=C(CH₃)- and
D is the group 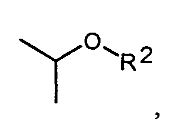
R¹ is an amino sugar of the formula 1a and
R² is a sugar of the formula 2a
or, in the case (18) that
A-B is the group -HC=CH- and
D is as defined above,
R¹ is hydrogen and
R² is hydrogen.

16. Compound according to Claim 12, **characterized in that**
A-B is the group -HC=CH-,
D is the group 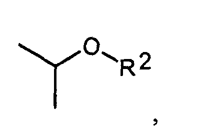
R¹ is an amino sugar of the formula 1a and
R² is a sugar of the formula 2a,
or in which
A-B is the group -HC=CH- or -HC=C(CH₃)-,
D is as defined above,
R¹ is hydrogen and
R² is a sugar of the formula 2a,
or in which
A-B is a group -HC=CH-,
D is as defined above,
R¹ is hydrogen and
R² is hydrogen.

17. Use of compounds according to any of Claims 11 to 16 for producing novel spinosyn derivatives.

## Revendications

1. Procédé de production de composés de formule générale (I) 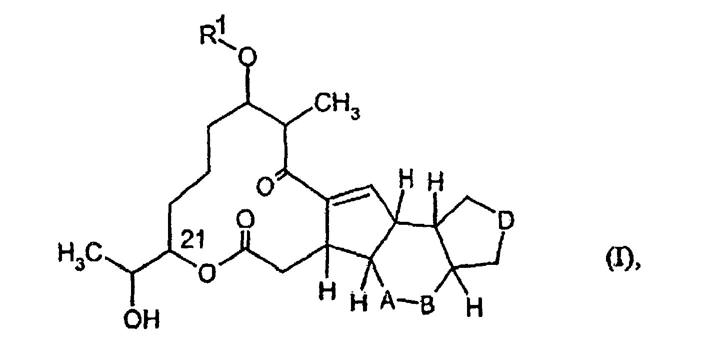 dans laquelle
A-B représente l'un des groupes suivants : -HC=CH-, -HC=C(CH₃)-, -H₂C-CH₂- ou -H₂C-CH(CH₃)-,
D représente le groupe 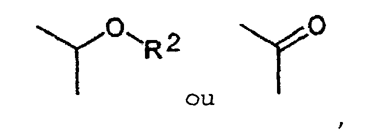
R¹ représente l'hydrogène ou un aminosucre et
R² représente l'hydrogène ou un sucre,
**caractérisé en ce qu'**on met en contact des composés de formule générale (II), 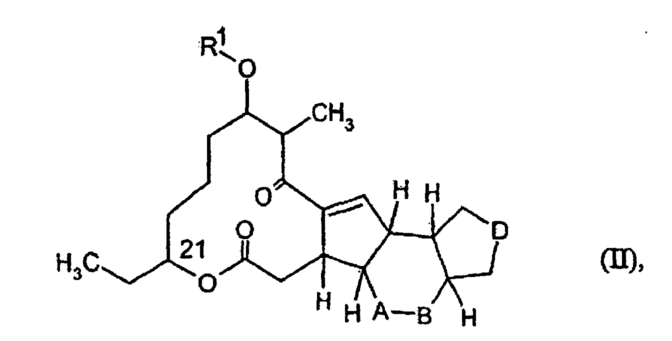 dans laquelle
A-B, D et R¹ ont les définitions indiquées ci-dessus,
avec un microorganisme dans un milieu nutritif aqueux dans des conditions aérobies ou avec un extrait enzymatique préparé à partir de ce microorganisme ou avec un ou plusieurs enzymes qui en sont isolées.

2. Procédé suivant la revendication 1, **caractérisé en ce qu'**on utilise des composés de formule générale (II), dans laquelle, au cas (1) où
A-B représente l'un des groupes suivants : -HC=CH-, -HC=C(CH₃)-, -H₂C-CH₂- ou -H₂C-CH(CH₃)- et
D représente le groupe 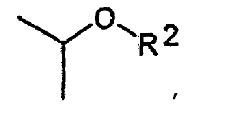
R¹ représente un aminosucre de formule 1a, 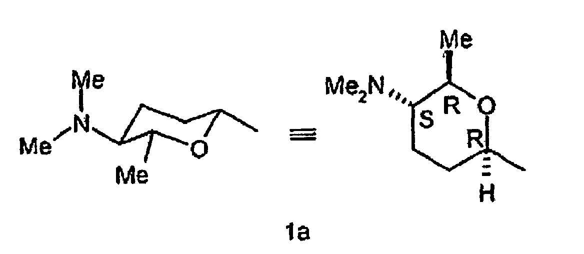 et
R² représente un sucre de formule 2a, 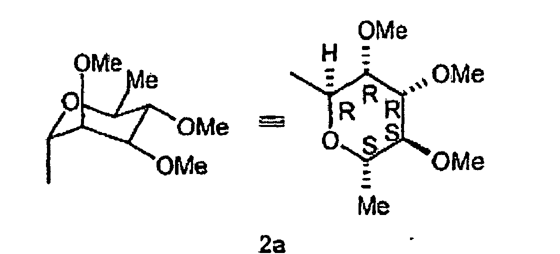
ou dans laquelle, au cas (2) où
A-B représente le groupe -HC=CH- ou -H₂C-CH₂- et
D a la définition indiquée ci-dessus,
R¹ représente un aminosucre de formule la indiquée ci-dessus et
R² représente l'hydrogène ou un sucre de formule 2b, 2c, 2d, 2e ou 2f, 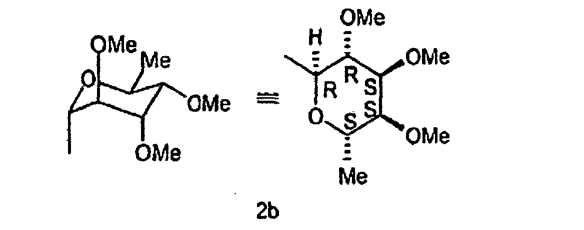 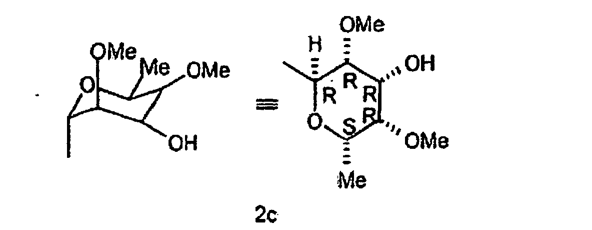 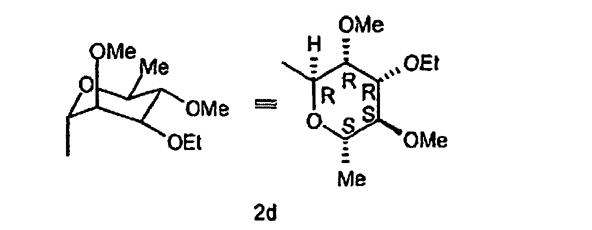 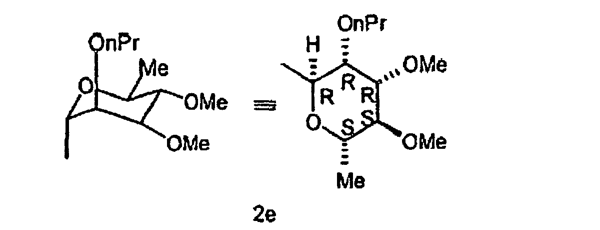 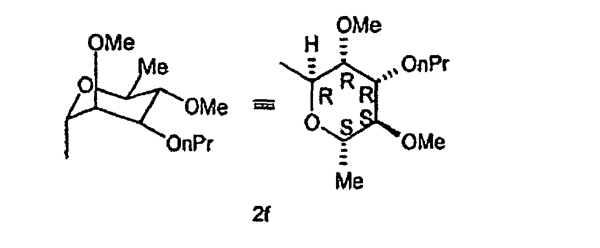
ou dans laquelle, au cas (3) où
A-B représente l'un des groupes suivants : -HC=CH-, -HC=C(CH₃)- ou -H₂C-CH₂- et
D a la définition indiquée ci-dessus,
R¹ représente l'hydrogène ou un aminosucre de formule 1b, 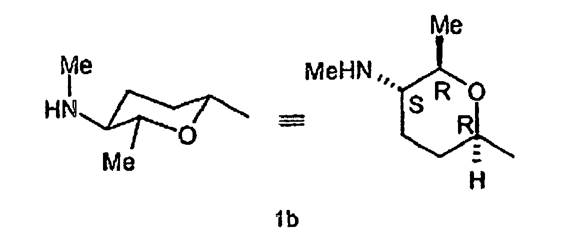
et
R² représente l'hydrogène ou un sucre de formule 2a mentionnée ci-dessus,
ou dans laquelle, au cas (4) où
A-B représente le groupe -HC=CH- ou -HC=C(CH₃)- et
D a la définition indiquée ci-dessus,
R¹ représente un aminosucre de formule 1a mentionnée ci-dessus et
R² représente un sucre de formule 2g, 2h, 2i, 2j ou 2k, 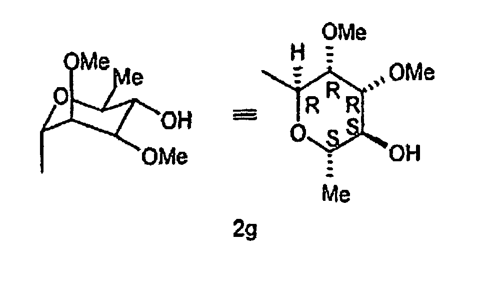 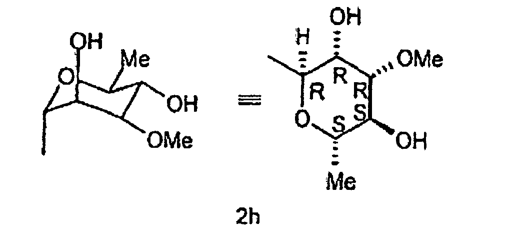 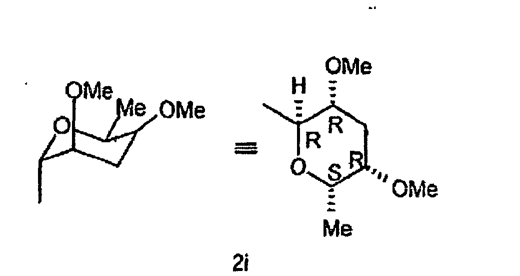 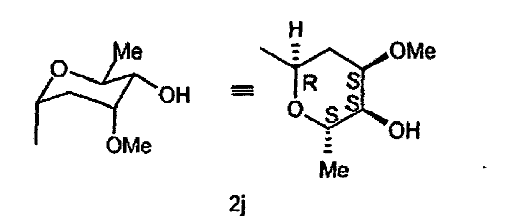 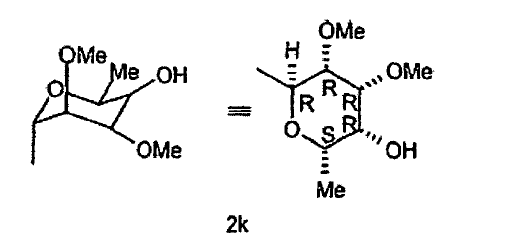
ou dans laquelle, au cas (5) où
A-B représente le groupe -HC=CH- ou -H₂C-CH₂- et
D a la définition indiquée ci-dessus,
R¹ représente un aminosucre de formule 1a mentionnée ci-dessus et
R² représente un sucre de formule 2l ou 2m, 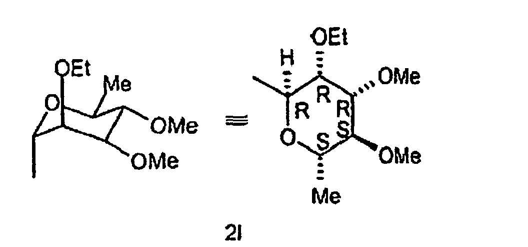 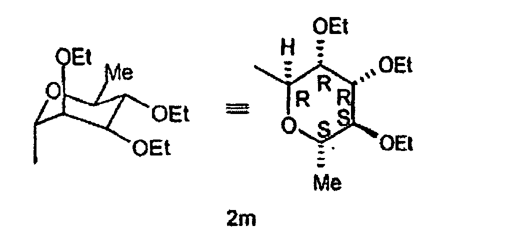
ou dans laquelle, au cas (6) où
A-B représente le groupe -HC=CH- ou -HC=C (CH₃) - et
D a la définition indiquée ci-dessus,
R¹ représente l'hydrogène ou un aminosucre de formule 1b mentionnée ci-dessus ou un aminosucre de formule 1c, 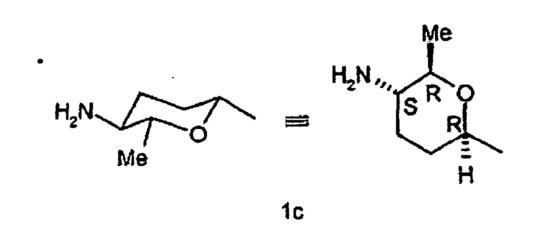
et
R² représente un sucre de formule 2b, 2c, 2g ou 2h mentionnée ci-dessus ou un sucre de formule 2n, 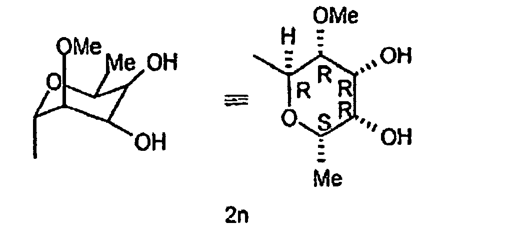
ou dans laquelle, au cas (7) où
A-B représente le groupe -HC=CH- et
D a la définition indiquée ci-dessus,
R¹ représente un aminosucre de formule 1a mentionnée ci-dessus et
R² représente un sucre de formule 2o, 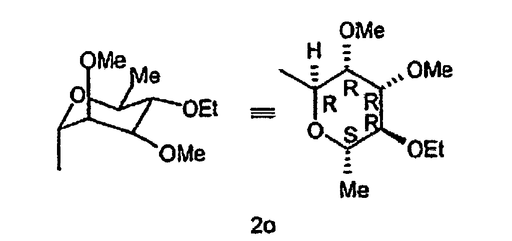
ou dans laquelle, au cas (8) où
A-B représente le groupe -HC=CH- et
D a la définition indiquée ci-dessus,
R¹ représente un aminosucre de formule 1b mentionnée ci-dessus et
R² représente un sucre de formule 2d, 2i ou 2j mentionnée ci-dessus ou un sucre de formule 2p, 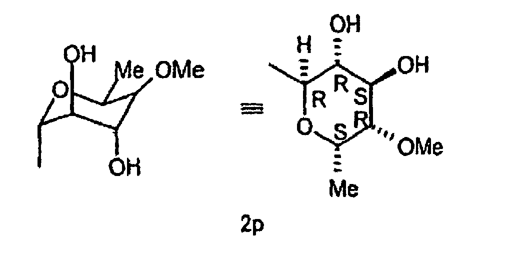
ou dans laquelle, au cas (9) où
A-B représente le groupe -HC=CH- et
D a la définition mentionée ci-dessus,
R¹ représente l'hydrogène ou un aminosucre de formule 1c mentionnée ci-dessus et
R² représente un sucre de formule 2i ou 2p mentionnée ci-dessus,
ou dans laquelle, au cas (10) où
A-B représente le groupe -HC=CH- et
D a la définition mentionnée ci-dessus,
R¹ représente un aminosucre de formule 1d, 1e ou 1f, 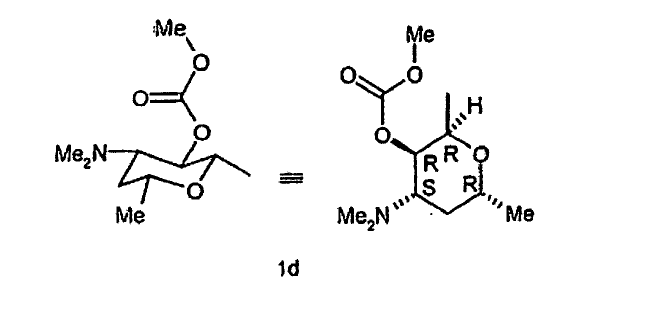 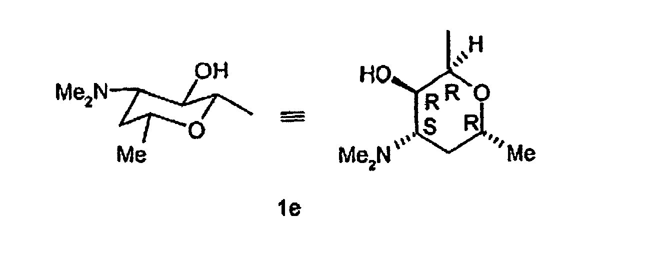 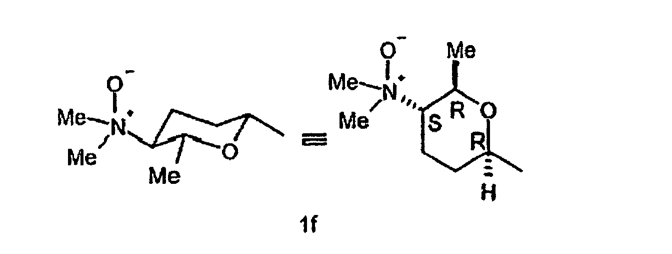
et
R² représente un sucre de formule 2a mentionnée ci-dessus,
ou dans laquelle, au cas (11) où
A-B représente le groupe -HC=CH- et
D représente le groupe 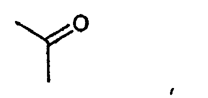
R¹ représente l'hydrogène ou un aminosucre de formule la mentionnée ci-dessus.

3. Procédé suivant la revendication 1 ou 2, **caractérisé en ce qu'**on utilise des composés de formule générale (II)
dans laquelle au cas (12) où
A-B représente le groupe -HC=CH- ou -HC=C(CH₃)- et
D représente le groupe 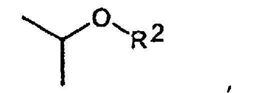
R¹ représente un aminosucre de formule 1a et
R² représente un sucre de formule 2a, 2g ou 2h,
ou dans laquelle, au cas (13) où
A-B représente le groupe -HC=CH- et
D a la définition mentionnée ci-dessus,
R¹ représente un aminosucre de formule 1a et
R² représente l'hydrogène ou un sucre de formule 2d, 2e, 21, 2m ou 2o,
ou dans laquelle, au cas (14) où
A-B représente le groupe -HC=CH- ou -HC=C(CH₃)- et
D a la définition indiquée ci-dessus,
R¹ représente l'hydrogène ou un aminosucre de formule 1b et
R² représente l'hydrogène ou un sucre de formule 2a,
ou dans laquelle A-B, D et R¹ sont tels que définis dans le cas (11).

4. Procédé suivant l'une des revendications 1 à 3, **caractérisé en ce qu'**on utilise des composés de formule générale (II)
dans laquelle au cas (15) où
A-B représente le groupe -HC=CH- ou -HC=C(CH₃)- et
D représente le groupe 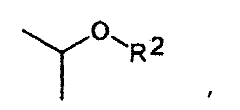
R¹ représente un aminosucre de formule 1a et
R² représente un sucre de formule 2a,
ou dans laquelle, au cas (16) où
A-B représente le groupe -HC=CH- et
D a la définition indiquée ci-dessus,
R¹ représente un aminosucre de formule 1a et
R² représente l'hydrogène ou un sucre de formule 2d, 2l ou 2m,
ou dans laquelle A-B, D et R¹ sont définis comme dans le cas (11).

5. Procédé suivant l'une des revendications 1 à 4, **caractérisé en ce qu'**on utilise des composés de formule (II) ,
dans laquelle, au cas (17) où
A-B représente le groupe -HC=CH- ou -HC=C(CH₃)- et
D est le groupe 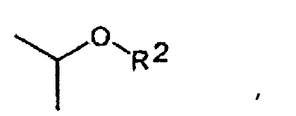
R¹ représente un aminosucre de formule 1a et
R² représente un sucre de formule 2a,
ou dans laquelle, au cas (18) où
A-B représente le groupe -HC=CH- et
D a la définition indiquée ci-dessus,
R¹ est l'hydrogène et
R² est l'hydrogène.

6. Procédé suivant l'une des revendications 1 à 5, **caractérisé en ce qu'**on utilise comme microorganisme un microorganisme du groupe des actinomycètes.

7. Procédé suivant la revendication 6, **caractérisé en ce qu'**on utilise comme microorganisme du groupe des actinomycètes un microorganisme du genre Streptomyces.

8. Procédé suivant la revendication 7, **caractérisé en ce qu'**on utilise comme microorganisme du genre Streptomyces une souche de l'espèce Streptomyces djakartensis, Streptomyces griseofuscus, Streptomyces caelestis, Streptomyces antibioticus, Streptomyces griseus ou Streptomyces aureofaciens.

9. Procédé suivant la revendication 8, **caractérisé en ce que** la souche représente les particularités caractérisantes des souches Streptomyces djakartensis DSM 14327, Streptomyces griseofuscus DSM 14330, Streptomyces caelestis DSM 14328, Streptomyces antibioticus DSM 14329, Streptomyces griseus DSM 14331 ou Streptomyces aureofaciens DSM 14332.

10. Procédé suivant l'une des revendications 1 à 9, **caractérisé en ce qu'**on isole les composés de formule générale (I).

11. Composés de formule générale (I), 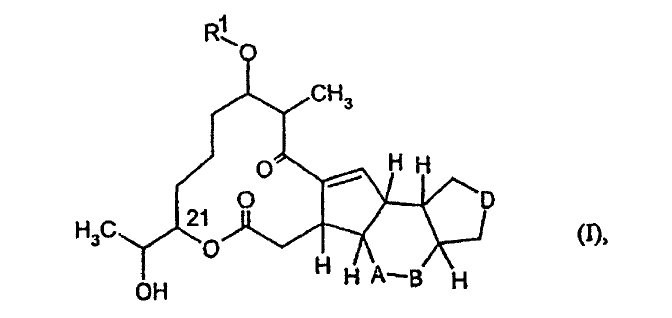 dans laquelle
A-B représente l'un des groupes suivants : -HC=CH-, -HC=C(CH₃)-, -H₂C-CH₂- ou -H₂C-CH(CH₃)-,
D est le groupe 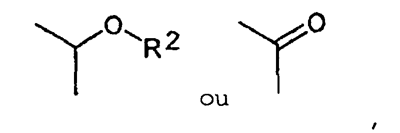
R¹ représente un aminosucre et
R² représente un sucre.

12. Composés suivant la revendication 11, **caractérisés en ce que**
au cas (1) où
A-B représente l'un des groupes suivants : -HC=CH-, -HC=C(CH₃)-, -H₂C-CH₂- ou -H₂C-CH(CH₃)- et
D représente le groupe 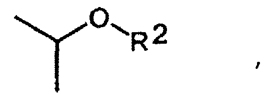
R¹ est un aminosucre de formule 1a, 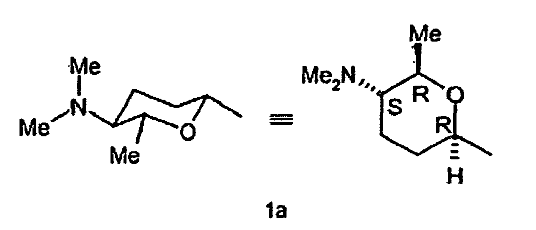 et
R² est un sucre de formule 2a, 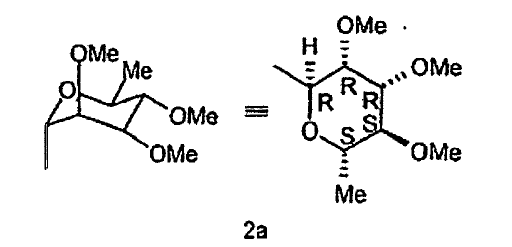
ou au cas (2) où
A-B représente le groupe -HC=CH- ou -H₂C-CH₂ et
D a la définition indiquée ci-dessus,
R¹ représente un aminosucre de formule 1a mentionnée ci-dessus et
R² représente l'hydrogène ou un sucre de formule 2b, 2c, 2d, 2e ou 2f, 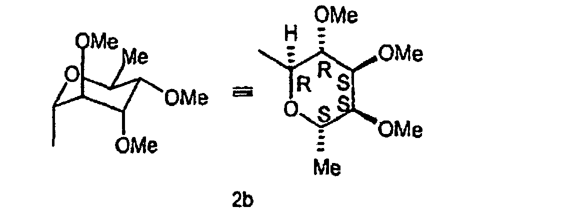 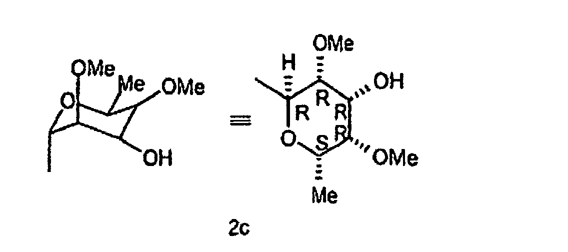 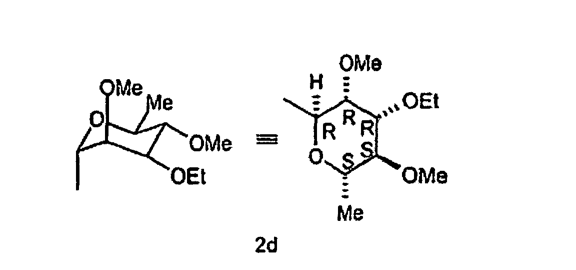 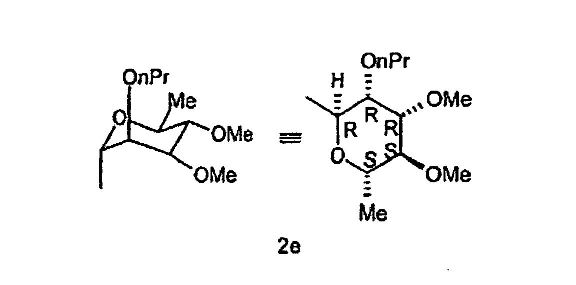 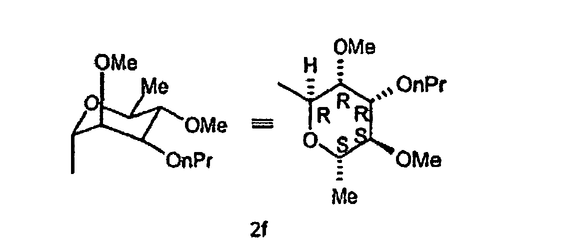
ou au cas (3) où
A-B représente l'un des groupes suivants : -HC=CH-, -HC=C(CH₃)- ou -H₂C-CH₂- et
D a la définition mentionnée ci-dessus,
R¹ représente l'hydrogène ou un aminosucre de formule 1b, 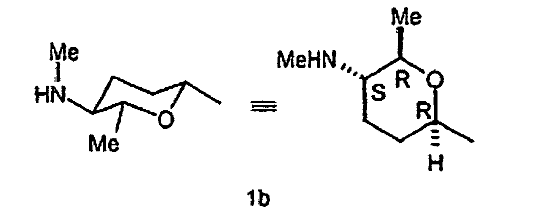 et
R² représente l'hydrogène ou un sucre de formule 2a mentionnée ci-dessus,
ou au cas (4) où
A-B représente le groupe -HC=CH- ou -HC=C(CH₃)- et
D a la définition mentionnée ci-dessus,
R¹ représente un aminosucre de formule 1a mentionnée ci-dessus et
R² représente un sucre de formule 2g, 2h, 2i, 2j ou 2k, 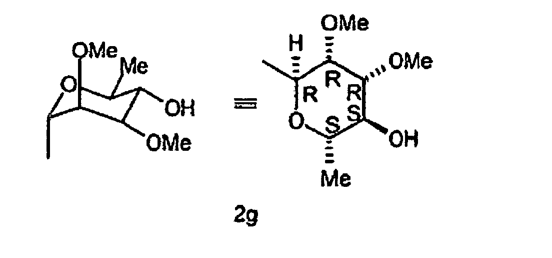 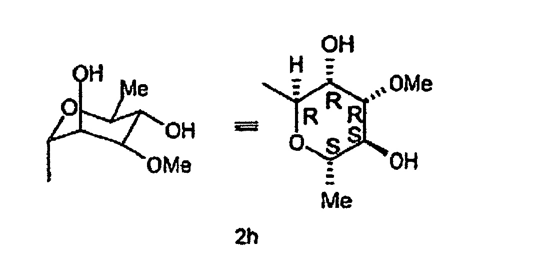 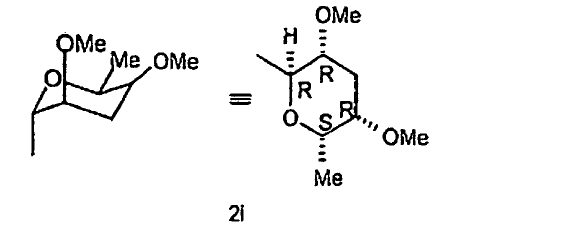 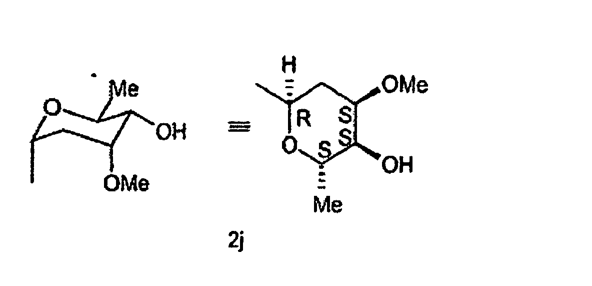 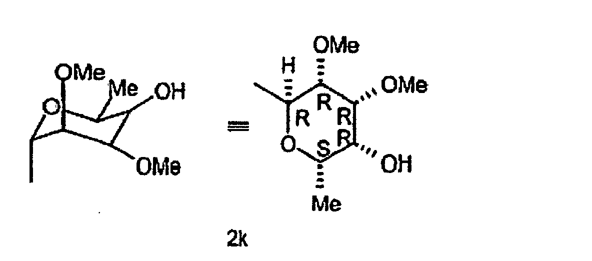
ou au cas (5) où
A-B représente le groupe -HC=CH- ou -H₂C-CH₂- et
D a la définition mentionnée ci-dessus,
R¹ représente un aminosucre de formule 1a mentionnée ci-dessus et
R² représente un sucre de formule 2l ou 2m, 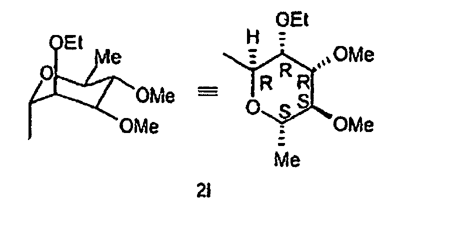 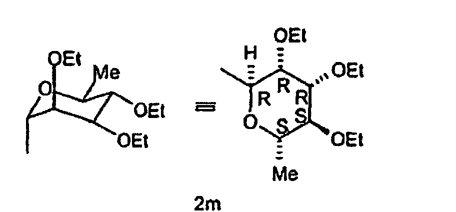
ou au cas (6) où
A-B représente le groupe -HC=CH- ou -HC=C(CH₃)- et
D a la définition mentionnée ci-dessus,
R¹ représente l'hydrogène ou un aminosucre de formule 1b mentionnée ci-dessus ou un aminosucre de formule 1c, 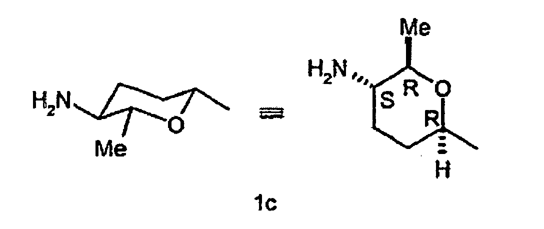 et
R² représente un sucre de formule 2b, 2c, 2g ou 2h mentionnée ci-dessus ou un sucre de formule 2n, 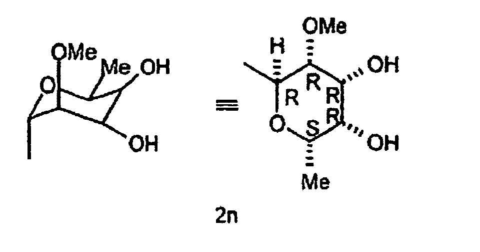
ou au cas (7) où
A-B représente le groupe -HC=CH- et
D a la définition indiquée ci-dessus,
R¹ représente un aminosucre de formule 1a mentionnée ci-dessus et
R² représente un sucre de formule 2o, 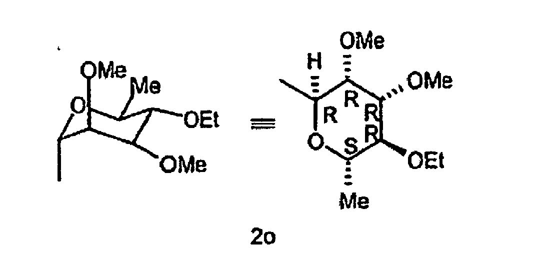
ou au cas (8) où
A-B représente le groupe -HC=CH- et
D a la définition mentionnée ci-dessus,
R¹ représente un aminosucre de formule 1b mentionnée ci-dessus et
R² représente un sucre de formule 2d, 2i ou 2j mentionnée ci-dessus,
ou un sucre de formule 2p, 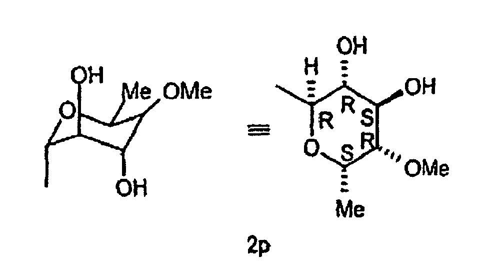
ou au cas (9) où
A-B représente le groupe -HC=CH- et
D a la définition indiquée ci-dessus,
R¹ représente l'hydrogène ou un aminosucre de formule 1c mentionnée ci-dessus et
R² représente un sucre de formule 2i ou 2p mentionnée ci-dessus,
ou au cas (10) où
A-B représente le groupe -HC=CH- et
D a la définition mentionnée ci-dessus,
R¹ représente un aminosucre de formule 1d, 1e mentionnée ci-dessus ou un aminosucre de formule 1f, 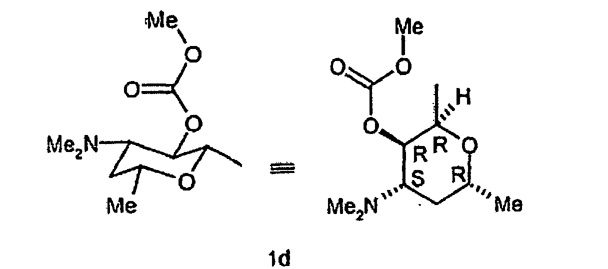 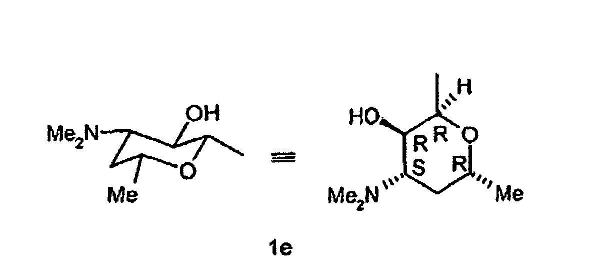 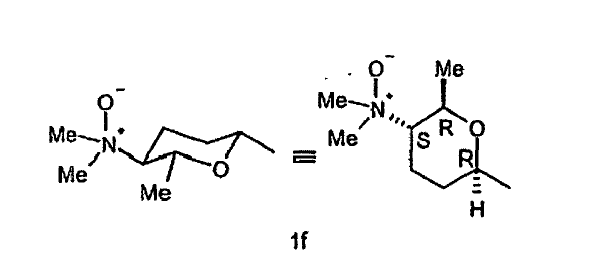
et
R² représente un sucre de formule 2a mentionnée ci-dessus,
ou au cas (11) où
A-B représente le groupe -HC=CH- et
D représente le groupe 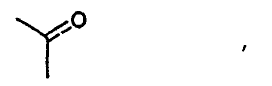
R¹ représente l'hydrogène ou un aminosucre de formule 1a mentionnée ci-dessus.

13. Composés suivant la revendication 12, **caractérisés en ce que**
au cas (12) où
A-B représente le groupe -HC=CH- ou -HC=C(CH₃)- et
D est le groupe 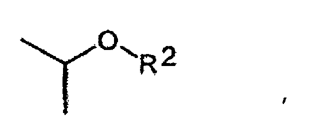
R¹ représente un aminosucre de formule 1a et
R² représente un sucre de formule 2a, 2g ou 2h,
ou au cas (13) où
A-B représente le groupe -HC=CH- et
D a la définition indiquée ci-dessus,
R¹ représente un aminosucre de formule 1a et
R² représente l'hydrogène ou un sucre de formule 2d, 2e, 21, 2m ou 2o,
ou au cas (14) où
A-B représente le groupe -HC=CH- ou -HC=C(CH₃)- et
D a la définition indiquée ci-dessus,
R¹ représente l'hydrogène ou un aminosucre de formule 1b et
R² représente l'hydrogène ou un sucre de formule 2a, ou bien A-B, D et R¹ sont définis comme dans le cas (11).

14. Composés suivant la revendication 12, **caractérisés en ce que**
au cas (15) où
A-B représente le groupe -HC=CH- ou -HC=C(CH₃)- et
D est le groupe 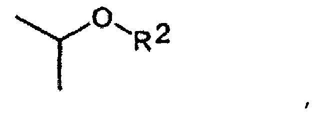
R¹ représente un aminosucre de formule 1a et
R² représente un sucre de formule 2a,
ou au cas (16) où
A-B représente le groupe -HC=CH- et
D a la définition mentionnée ci-dessus,
R¹ représente un aminosucre de formule 1a et
R² représente l'hydrogène ou un sucre de formule 2d, 2l ou 2m,
ou bien A-B, D et R¹ sont définis comme pour le cas (11).

15. Composés suivant la revendication 12, **caractérisés en ce que**
au cas (17) où
A-B représente le groupe -HC=CH- ou -HC=C(CH₃)- et
D est le groupe 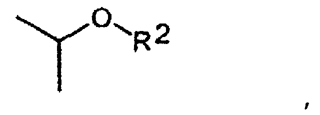
R¹ représente un aminosucre de formule 1a et
R² représente un sucre de formule 2a,
ou au cas (18) où
A-B représente le groupe -HC=CH- et
D a la définition indiquée ci-dessus,
R¹ représente l'hydrogène et
R² représente l'hydrogène.

16. Composés suivant la revendication 12, **caractérisés en ce que**
A-B représente le groupe -HC=CH-,
D est le groupe 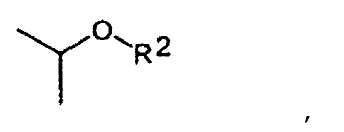
R¹ représente un aminosucre de formule 1a et
R² représente un sucre de formule 2a,
ou dans laquelle
A-B représente le groupe -HC=CH- ou -HC=C(CH₃)-,
D a la définition mentionnée ci-dessus,
R¹ représente l'hydrogène et
R² représente un sucre de formule 2a,
ou dans laquelle
A-B représente un groupe -HC=CH-,
D a la définition mentionnée ci-dessus,
R¹ représente l'hydrogène et
R² représente l'hydrogène.

17. Utilisation de composés suivant l'une des revendications 11 à 16 pour 1a préparation de nouveaux dérivés de spinosynes.
